# EUROPEAN PATENT SPECIFICATION

(11) **EP 1 330 652 B1**
(45) Date of publication and mention of the grant of the patent: **27.06.2007**
(21) Application number: 01982003.4
(22) Date of filing: 26.10.2001
(51) Int. Cl.: G01N 33/574, C07K 14/47, C12N 9/64

(54) **METHOD FOR DETECTING OVARIAN CANCER BASED ON HUMAN KALLIKREIN 6 (HK6)**
VERFAHREN ZUR ERKENNUNG VON EIERSTOCKKREBS MITTELS MENSCHLICHEM KALLIKREIN (HK6)
METHODE DE DETECTION DU CANCER DE L'OVAIRE A L'AIDE DE KALLIKREINE HUMAINE (HK6)

(30) Priority: 27.10.2000 US 243789 P
(43) Date of publication of application: 30.07.2003
(73) Proprietor: MOUNT SINAI HOSPITAL, Toronto Ontario M5G 1X5 (CA)
(72) Inventor: DIAMANDIS, Eleftherios, P., Toronto, Ontario M5G 2X2 (CA)
(74) Representative: Holliday, Louise Caroline
(86) International application number: PCT/CA2001/001505
(87) International publication number: WO 2002/035232

(56) References cited:
- WO-A-01/59158
- WO-A-98/11238
- WO-A-98/41656
- US-A- 5 660 827
- ANISOWICZ A ET AL: "A NOVEL PROTEASE HOMOLOG DIFFERENTIALLY EXPRESSED IN BREAST AND OVARIAN CANCER" MOLECULAR MEDICINE, BLACKWELL SCIENCE, CAMBRIDGE, MA, US, vol. 2, no. 5, 1 September 1996 (1996-09-01), pages 624-636, XP002060076 ISSN: 1076-1551 cited in the application
- YOUSEF G M ET AL: "The KLK7 (PRSS6) gene, encoding for the stratum corneum chymotryptic enzyme is a new member of the human kallikrein gene family - genomic characterization, mapping, tissue expression and hormonal regulation" GENE, ELSEVIER BIOMEDICAL PRESS. AMSTERDAM, NL, vol. 254, no. 1-2, 22 August 2000 (2000-08-22), pages 119-128, XP004208787 ISSN: 0378-1119
- ELEFTHERIOS P: "immunofluorometric assay of human kallikrein 6 (zyme/protease M/neurosin) and preliminary clinical applications." CLINICAL BIOCHEMISTRY, vol. 33, no. 5, July 2000 (2000-07), pages 369-375, XP002207083 cited in the application
- DIAMANDIS EP ET AL: "the new human kallikrein gene family: implications in carcinogenesis" TRENDS IN ENDOCRINOLOGY AND METABOLISM, vol. 11, no. 2, November 2000 (2000-11), pages 54-60, XP002207049 cited in the application

## Description

### FIELD OF THE INVENTION

The invention relates to diagnostic and prognostic methods for ovarian carcinoma.

### BACKGROUND OF THE INVENTION

Until recently, the human kallikrein gene family was thought to consist of only 3 genes: pancreatic/renal kallikrein (KLK1, encoding for hK1 protein), human glandular kallikrein 2 (KLK2, encoding for hK2 protein) and human kallikrein 3 (KLK3, encoding for hK3 protein or prostate-specific antigen, PSA). The latter two kallikreins, PSA and hK2, are relatively prostatic-specific and they have already found important applications as biomarkers for the diagnosis and monitoring of prostate cancer (1-6).

New members of the human kallikrein gene family have recently been discovered (1). This gene family now contains at least 14 genes which are all encoding for serine proteases, show significant homology at both the DNA and amino acid level and they are all localized at the chromosomal locus 19q13.3-q 13.4, in tandem, without any intervention from other non-kallikrein genes. This area of investigation has recently been reviewed (1).

The KLK6 gene (encoding for human kallikrein 6, hK6) has been cloned independently by three groups of investigators and was previously given the names zyme (7), protease M (8) and neurosin (9). Recently, uniform nomenclature for all newly discovered and the traditional kallikrein genes has been established (10). The KLK6 gene encodes for a trypsin-like serine protease of 244 amino acids in length, of which 16 amino acids constitute the signal peptide and 5 amino acids, the activation peptide. The mature enzyme consists of 223 amino acids. It has been previously predicted that hK6 is a secreted protein (7-9,11). This was recently verified by finding hK6 protein in various biological fluids, including cerebrospinal fluid, nipple aspirate fluid, breast cyst fluid, male and female serum, seminal plasma, amniotic fluid and breast cancer cytosols (12). Little et al. (7) have demonstrated that this enzyme has amyloidogenic potential in the brain and may play a role in the development and progression of Alzheimer's disease. Others have cloned the same gene by the method of differential display, and found that it is down-regulated in aggressive forms of breast cancer (8). The same gene was cloned by Yamashiro et al. from the human colon adenocarcinoma cell line COLO 201 (9).

Among the classical human kallikreins, PSA has proven to be the most valuable biomarker for prostate cancer and is currently used for diagnosis and monitoring of this disease (2-4). Another potential prostatic biomarker, hK2, has also been recently introduced (5, 6). Among the newly discovered kallikreins (1), none of them has been examined as a serological marker for any malignancy since no methods currently exist to measure the secreted proteins with high sensitivity and specificity.

Ovarian cancer is a serious disease which causes more deaths than any other cancer of the female reproductive system (13). Since survival could be dramatically improved if the disease is diagnosed early (14), there is great interest in the identification of biomarkers that could aid in the early detection and facilitate grading and/or staging (15). Unfortunately, the current serological markers for ovarian carcinoma, including CA125 (16-19), inhibin (20-23), OVX1 (24) as well as many other markers (reviewed in 25) have shown some promise but have not gained wide clinical acceptance. Another potential ovarian cancer marker, lysophosphatidic acid, appears to also have some value for this purpose (26).

Anisowiez et al, Molecular Medicine (1996), 2(5), 624-636 relates to the isolation of protease M (also known as kallikrein 6) and discloses that protease M mRNA is strongly expressed in ovarian cancer tissue and cell lines and may be useful as a marker for detection of primary ovarian cancer. However, the authors concluded that the protein is primarily localized intracellularly rather than secreted as they were able to detect it in cell lysates but not in conditioned media. The authors also observed a lack of correlation between protease M mRNA expression levels (high) and protease M protein levels (low or absent) in breast cancer cell lines.

WO-A-98/11238 relates to the discovery of protease M and discloses the use of antibodies to detect protease M in a cell lysate or supernatant.

WO-A-98/41656 relates to a method for detecting ovarian cancer which comprises screening for a plurality of protease markers in tissue. In particular, the document relates to detecting in tissue samples proteases from the group consisting of Stratum Corneum Chymotrypsin Enzyme (SCCE), TADG12, TADG13, TADG14, Hepsin, Pump-1 and Protease M.

There is an urgent need for discovery and validation of new biomarkers for ovarian carcinoma- Early diagnosis of ovarian cancer, particularly with serological analysis, may improve clinical outcomes through administration of effective treatment.

### SUMMARY OF THE INVENTION

A highly sensitive hK6 immunoassay for the measurement of hK6 in various biological fluids was developed (Example 1). Using this sensitive assay, the hK6 concentration in serum was found to be significantly increased in a large proportion of patients with ovarian cancer. In particular, hK6 was found to be significantly increased in ovarian cancer patients when compared to normal non-cancer patients and patients with benign disease. Thus hK6 constitutes a new biomarker for diagnosis and monitoring of ovarian cancer. hK6 may be used to diagnose and monitor late stage ovarian cancer, and it may be used as a biomarker before surgery or after relapse.

The present inventors also quantitated the amount of hK6 in extracts of ovarian tumors and determined that the amount of hK6 correlated with clinicopathological variables documented at the time of surgical excision and with progression free survival and overall survival. Increased hK6 levels were found to be predictive of more aggressive tumor behaviour over time. hK6 positivity was found to be associated with about a 2-fold increase in the risk of both disease progression and of death.

hK6, and agents that bind to hK6 may be used to detect ovarian cancer and in particular they can be used in the diagnostic evaluation of ovarian cancer and the identification of subjects with a predisposition to ovarian cancer.

The present invention relates to an *in vitro* method for diagnosing or monitoring ovarian cancer or determining the prognosis of ovarian cancer in a subject comprising:
(a) determining the level of human kallikrein 6 in a serum sample from the subject; and
(b) comparing the determined level of human kallikrein 6 to a level present in a control serum sample acquired from a control subject known not to be inflicted by ovarian cancer or with benign disease or a control serum sample taken from the subject at a different time, wherein an increase in the level of human kallikrein 6 in the serum sample from the subject as compared to the level of human kallikrein 6 in the control serum sample is indicative of ovarian cancer in the subject or poor prognosis.

The terms "detecting" or "detect" include assaying, quantitating, imaging or otherwise establishing the presence or absence of the target hK6, subunits thereof, or combinations or reagent bound targets, and the like, or assaying for, imaging ascertaining, establishing, or otherwise determining one or more factual characteristics of ovarian cancer, metastasis, stage, or similar conditions. The term encompasses diagnostic, prognostic, and monitoring applications for hK6.

In an embodiment, the invention relates to a method for detecting ovarian cancer in a subject by quantitating hK6 in a biological sample from the subject comprising (a) reacting the biological sample with an antibody specific for hK6 which is directly or indirectly labelled with a detectable substance; and (b) detecting the detectable substance.

The invention further relates to a method for diagnosing and monitoring ovarian carcinoma in a subject by quantitating hK6 in a sample from a subject comprising (a) reacting a biological sample from the subject with an antibody specific for hK6 which is directly or indirectly labelled with a detectable substance; (b) and detecting the detectable substance.

Embodiments of the methods of the invention involve (a) reacting a biological sample from a subject with an antibody specific for hK6 which is directly or indirectly labelled with an enzyme; (b) adding a substrate for the enzyme wherein the substrate is selected so that the substrate, or a reaction product of the enzyme and substrate forms fluorescent complexes; (c) quantitating hK6 in the sample by measuring fluorescence of the fluorescent complexes; and (d) comparing the quantitated levels to that of a standard. The standard may correspond to levels obtained for other samples from the subject patient, or control subjects. In an embodiment the quantitated levels are compared to levels quantitated for subjects without ovarian carcinoma wherein an increase in hK6 levels compared with the control subjects is indicative of ovarian carcinoma, in particular late stage ovarian carcinoma.

A preferred embodiment of the invention comprises the following steps
(a) incubating a biological sample with a first antibody specific for hK6 which is directly or indirectly labeled with a detectable substance, and a second antibody specific for hK6 which is immobilized;
(b) separating the first antibody from the second antibody to provide a first antibody phase and a second antibody phase;
(c) detecting the detectable substance in the first or second antibody phase thereby quantitating hK6 in the biological sample; and
(d) comparing the quantitated hK6 with levels for a standard.

The standard may correspond to levels quantitated for samples from healthy control subjects, from subjects with benign disease, subjects with early stage disease, or from other samples of the subject. Increased levels of hK6 as compared to the standard may be indicative of ovarian cancer, in particular late stage ovarian cancer.

The invention also contemplates the methods described herein using multiple markers for ovarian cancer. Therefore, the invention contemplates a method for anaylzing a biological sample for the presence of hK6 and other markers that are specific indicators of ovarian cancer. Other markers include markers to kallikreins such as human stratum corneum chymotryptic enzyme (HSCCE), kallikrein 4, kallikrein 5, kallikrein 8, kallikrein 9, kallikrein 10, kallikrein 11; CA125, CA15-3, CA19-9, OVX1, lysophosphatidic acid (LPA) and carcinoembryonic antigen (CEA). Preferably the other markers are markers to kallikreins. In a preferred embodiment, the markers are two or more of hK6, MK10, and CA 125. The methods described herein may be modified by including reagents to detect the additional markers, or nucleic acids for the markers.

The invention also relates to kits for carrying out the methods of the invention.

Other objects, features and advantages of the present invention will become apparent from the following detailed description. It should be understood, however, that the detailed description and the specific examples while indicating preferred embodiments of the invention are given by way of illustration only, since various changes and modifications within the spirit and scope of the invention will become apparent to those skilled in the art from this detailed description.

### DESCRIPTION OF THE DRAWINGS

The invention will now be described in relation to the drawings in which:
Figure 1 is a calibration curve for a hK6 protein assay. The fluorescence of the zero standard (-18,000 arbitrary fluorescence units) was subtracted from all other measurements.
Figure 2 shows the results of high performance liquid chromatography separation of three biological fluids and analysis of all fractions with the developed hK6 immunoassay. In all three fluids, a single immunoreactive peak around fractions 38 - 42 was detected, corresponding to a molecular mass of ~ 30 kDa. The column was calibrated with molecular weight standards (shown on top with arrows; masses are in kDa). The milk sample was diluted 10 times before injection into the HPLC column.
Figure 3 is a graph showing the results of the analysis of various human tissue cytosolic extracts for hK6 protein
Figure 4 is a graph showing the frequency distribution of hK6 concentrations in the serum of 80 patients with ovarian carcinoma. The level of 15 µg/L which was used as a cutoff in Table 1 is indicated by an arrow. About 66% of ovarian cancer patients have serum hK6 concentration higher than this cutoff value. From another 298 serum samples with non-ovarian cancer, only 2 sera had values slightly higher than 15 µg/L (see Table 1).
Figure 5 is a graph showing the correlation between hK6 and CA125 concentration in 96 serum samples from ovarian cancer patients.
Figure 6 are graphs showing the analysis of hK6 and CA125 in serial serum samples from patients with ovarian cancer. These data suggest that hK6 may have value for patient monitoring.
Figure 7A is a graph showing the distribution of hK6 in normal, to benign, to cancer patients.
Figure 7B is a graph showing the distribution of CA 125 in normal, to benign, to cancer patients.
Figure 8 is a graph showing the concentration of hK6 in pre-surgical and post-surgical serum samples of ovarian cancer patients.
Figure 9 is a graph showing the correlation between serum hK6 and CA125 concentrations.
Figure 10 is a graph showing the sensitivity and specificity of serum hK6 concentrations.
Figure 11A is a graph showing hK6 concentration versus stage of ovarian cancer.
Figure 11B is a graph showing hK6 concentration versus grade of ovarian cancer.
Figure 12A is a graph showing the survival probability versus progression -free survival (PFS).
Figure 12B is a graph showing the survival probability versus overall survival (OS).

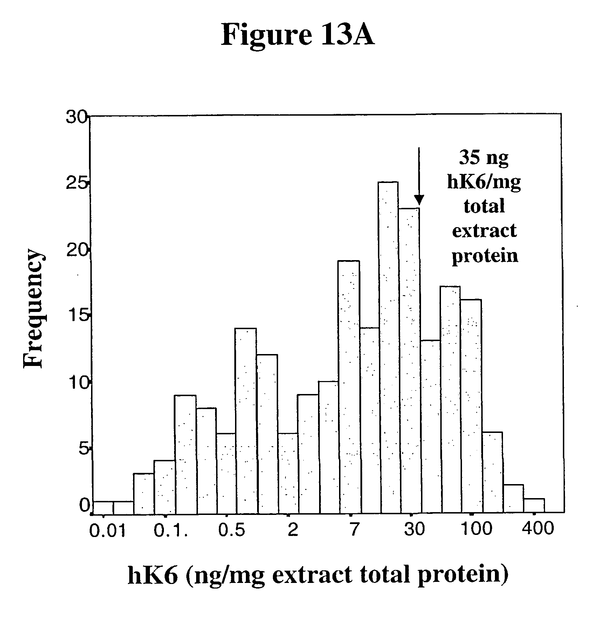

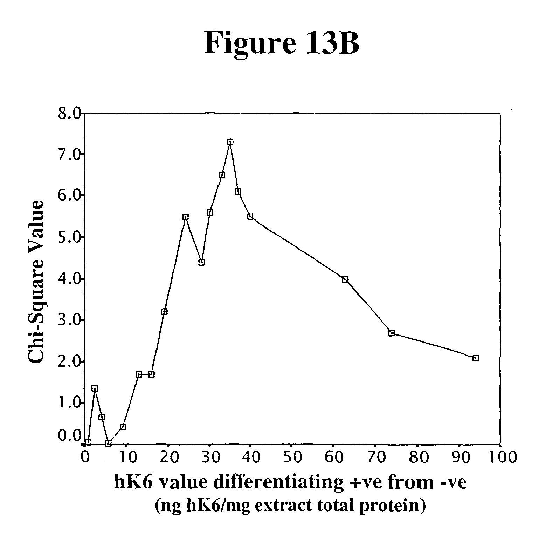

Figure 13(A) is a graph showing the frequency distribution of hK6 specific activity in ovarian tumor extracts. The value of 35ng/mg of total protein corresponds to the limit that, according to Chi square analysis, gives the best prediction of overall survival of the study population. (See Figure 13(B) for Chi square plot.) Tumors with hK6 in excess of 35 ng/mg total protein were classified as hK6 positive and those with values less than or equal to 35 ng/mg total protein were classified as hK6 negative. 30% of the tumors were classified as positive by this criterion. (B) Plot of hK6 tumor specific activity versus Chi-square statistic to determine the limit between hK6 positive and hK6 negative tumors that is most predictive of overall survival. Maximum predictive potential occurred between 28 to 40 ng hK6 total extract protein with a peak at 35 ng hK6/mg total extract protein.
Figure 14 is a graph showing a comparison of hK6 concentration in extracts from normal ovarian tissues ("normal"), and ovarian cancer ("cancer"). N indicates the number of specimens in each group. Horizontal bars represent the median hK6 specific activity (ng hK6/mg total extract protein) in each group. The Krustal Wallis test showed that extracted hK6 specific activity was significantly elevated in the ovarian tumor preparations (P<0.001).
Figure 15 is a graph showing the distribution of hK6 specific activity (ng hK6/mg total protein) in tumor extracts from stage I/II and stage III/IV ovarian cancer patients. N indicates the number of tumors comprising each group. Horizontal bars represent the median value of hK6 tumor specific activity. The Mann-Whitney test demonstrated that hK6 specific activity was significantly elevated in tumors from patients with stage III/IV ovarian cancer (P=0.002).
Figure 16 shows Kaplan-Meier survival curves of the entire patient population under study:effect of hK6 status. Top: progression-free survival (PFS). Bottom: overall survival (OS). The patient number in each group (n) is indicated as is the statistical significance (P value) of the survival difference between hK6 positive and hK6 negative groups. The adverse effect of hK6 positivity on both time to progression and overall survival was significant.
Figure 17 are graphs showing the effect of hK6 status (positive or negative) on progression -free survival (PFS) and on overall survival (OS) among patients with Grade I and II ovarian tumor. The patient number in each group (n) is indicated as is the statistical significance (P value) of the survival difference between hK6 positive and hK6 negative individuals. The adverse effect of hK6 positivity both on time to progression and on overall survival was significant (P≤0.002).

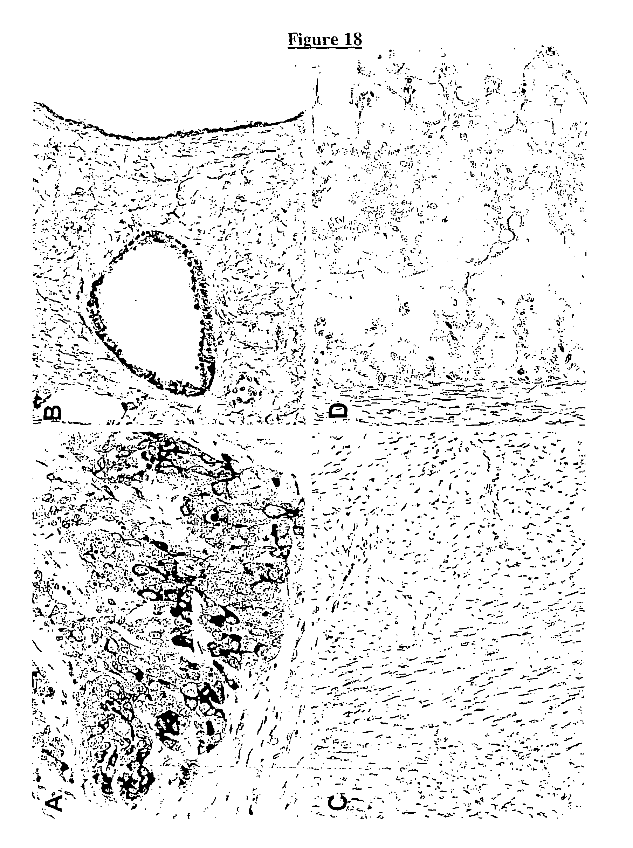

Figure 18 is a blot showing immunohistochemical localization of hK6 in ovarian neoplasms of varying malignant potential, cell type, and origin (epithelial versus mesenchymal). (A) Invasive papillary serous adenocarcinoma, the common malignant epithelial tumor of the ovary. Note strong cytoplasmic staining of many tumor cells, and absence of any staining of stroma or vessels. (B) Serous cystadenofibroma, a benign, mixed epithelial and fibrous neoplasm. Innumostaining is absent in the fibrous component, but strongly positive in the cytoplasm of the epithelium lining the cysts. (C) Ovarian leiomyoma, a benign smooth muscle tumor. Note the absence of staining. (D) Mucinous epithelial tumor of low malignant potential, an epithelial tumor of intermediate grade. Note weak, diffuse cytoplasmic staining of neoplastic epithelium and absent staining in supportive stroma (far left).

### DETAILED DESCRIPTION OF THE INVENTION

As hereinbefore mentioned, the present invention provides a method for monitoring, diagnosing, or for the prognosis of ovarian carcinoma in a subject by detecting hK6 in a biological sample from the subject. In an embodiment, the method comprises reacting the sample with an agent that binds to hK6, preferably an antibody specific for hK6 which is directly or indirectly labelled with a detectable substance, and detecting the detectable substance.

The methods of the invention may be used for the detection of either an over- or an under-abundance of hK6 relative to a non- disorder state or the presence of a modified (e.g., less than full length) hK6 which correlates with a disorder state (e.g ovarian cancer), or a progression toward a disorder state. The methods described herein may be used to evaluate the probability of the presence of malignant or pre-malignant cells, for example, in a group of cells freshly removed from a host. Such methods can be used to detect tumors, quantitate their growth, and help in the diagnosis and prognosis of disease. The methods can be used to detect the presence of cancer metastasis, as well as confirm the absence or removal of all tumor tissue following surgery, cancer chemotherapy, and/or radiation therapy. They can further be used to monitor cancer chemotherapy and tumor reappearance.

The methods of the invention are particularly useful in the diagnosis of late stage ovarian carcinoma and for the prognosis of ovarian carcinoma disease progression and mortality. As illustrated herein increased levels of hK6 detected in serum compared to a standard are indicative of late stage disease, and increased levels of hK6 in tumor tissues or extracts thereof compared to a standard are indicative of increased risk of disease progression and mortality.

The terms "sample", "biological sample", and the like mean a material known to or suspected of expressing or containing hK6. The test sample can be used directly as obtained from the source or following a pretreatment to modify the character of the sample. The sample can be derived from any biological source, such as tissues or extracts, including cells (e.g. tumor cells) and physiological fluids, such as, for example, whole blood, plasma, serum, saliva, ocular lens fluid, cerebral spinal fluid, sweat, urine, milk, ascites fluid, synovial fluid, peritoneal fluid and the like. The sample can be obtained from animals, preferably mammals, most preferably humans. The sample can be treated prior to use, such as preparing plasma from blood, diluting viscous fluids, and the like. Methods of treatment can involve filtration, distillation, extraction, concentration, inactivation of interfering components, the addition of reagents, and the like. Proteins may be isolated from the samples and utilized in the methods of the invention. In a preferred embodiment, the biological sample is serum or tumor tissue extracts, most preferably serum.

In embodiments of the invention, the method described herein is adapted for diagnosing and monitoring, and for the prognosis of ovarian carcinoma by detecting hK6 in biological samples from a subject. These applications require that the amount of hK6 detected in a sample from a subject being tested be compared to levels detected for another sample or an earlier sample from the subject, or levels detected for a control sample. Levels for control samples from healthy subjects or subjects with benign disease may be established by prospective and/or retrospective statistical studies. Healthy subjects who have no clinically evident disease or abnormalities may be selected for statistical studies. Diagnosis may be made by a finding of statistically different levels of hK6 compared to a control sample or previous levels detected for the same subject.

The term "hK6" refers to human kallikrein 6, (also known as zyme, protease M, and neurosin) a trypsin-like serine protease of 244 amino acids in length, of which 16 amino acids constitute the signal peptide and 5 amino acids, the activation peptide (7, 8, and 9). The term includes all homologs, naturally occurring allelic variants, isoforms and precursors of human kallikrein 6 of GenBank Accession Nos. AF013988, AF149289, HSU62801, D78203, and NM002774. In general for example, naturally occurring allelic variants of human kallikrein 6 will share significant homology (70-90%) to the sequences shown in GenBank Accession Nos. AF013988, AF149289, HSU62801, D78203, and NM002774. Allelic variants may contain conservative amino acid substitutions from the KLK6 sequence or will contain a substitution of an amino acid from a corresponding position in a hK6 homologue such as, for example, the murine kallikrien 6 homologue.

The term "subject" refers to a warm-blooded animal such as a mammal which is afflicted with or suspected to be afflicted with ovarian cancer. Preferably, "subject" refers to a human.

The antibodies specific for hK6 used in the methods of the invention may be obtained from scientific or commercial sources. Alternatively, isolated native hK6 or recombinant hK6 may be utilized to prepare antibodies, monoclonal or polyclonal antibodies, and immunologically active fragments (e.g. a Fab or (Fab)₂ fragment), an antibody heavy chain, an antibody light chain, humanized antibodies, a genetically engineered single chain Fᵥ molecule (Ladner et al, U.S. Pat. No. 4,946,778), or a chimeric antibody, for example, an antibody which contains the binding specificity of a murine antibody, but in which the remaining portions are of human origin. Antibodies including monoclonal and polyclonal antibodies, fragments and chimeras, may be prepared using methods known to those skilled in the art. Preferably, antibodies used in the methods of the invention are reactive against hK6 if they bind with a Kₐ of greater than or equal to 10⁻⁷ M. In a sandwich immunoassay of the invention mouse polyclonal antibodies and rabbit polyclonal antibodies are utilized.

Antibodies specifically reactive with hK6, or derivatives, such as enzyme conjugates or labeled derivatives, may be used to detect hK6 in various biological samples, for example they may be used in any known immunoassays which rely on the binding interaction between an antigenic determinant of a protein and the antibodies. Examples of such assays are radioimmunoassays, enzyme immunoassays (e.g.ELISA), immunofluorescence, immunoprecipitation, latex agglutination, hemagglutination, and histochemical tests.

An antibody specific for hK6 may be labelled with a detectable substance and localised or identified in biological samples based upon the presence of the detectable substance. Examples of detectable substances include, but are not limited to, the following: radioisotopes (e.g., ³H, ¹⁴C, ³⁵S, ¹²⁵I, ¹³¹I), fluorescent labels (e.g., FITC, rhodamine, lanthanide phosphors), luminescent labels such as luminol; enzymatic labels (e.g., horseradish peroxidase, beta-galactosidase, luciferase, alkaline phosphatase, acetylcholinesterase), biotinyl groups (which can be detected by marked avidin e.g., streptavidin containing a fluorescent marker or enzymatic activity that can be detected by optical or calorimetric methods), predetermined polypeptide epitopes recognized by a secondary reporter (e.g., leucine zipper pair sequences, binding sites for secondary antibodies, metal binding domains, epitope tags). Indirect methods may also be employed in which the primary antigen-antibody reaction is amplified by the introduction of a second antibody, having specificity for the antibody reactive against hK6. By way of example, if the antibody having specificity against hK6 is a rabbit IgG antibody, the second antibody may be goat anti-rabbit gamma-globulin labelled with a detectable substance as described herein.

Methods for conjugating or labelling the antibodies discussed above may be readily accomplished by one of ordinary skill in the art. (See for example Inman, Methods In Enzymology, Vol. 34, Affinity Techniques, Enzyme Purification: Part B, Jakoby and Wichek (eds.), Academic Press, New York, p. 30, 1974; and Wilchek and Bayer, "The Avidin-Biotin Complex in Bioanalytical Applications,"Anal. Biochem. 171:1-32, 1988 re methods for conjugating or labelling the antibodies with enzyme or ligand binding partner).

Time-resolved fluorometry may be used to detect a signal. For example, the method described in Christopoulos TK and Diamandis EP Anal Chem 1992:64:342-346 may be used with a conventional time-resolved fluorometer.

Therefore, in accordance with an embodiment of the invention, a method is provided wherein a hK6 antibody is labelled with an enzyme, a substrate for the enzyme is added wherein the substrate is selected so that the substrate, or a reaction product of the enzyme and substrate, forms fluorescent complexes with a lanthanide metal. A lanthanide metal is added and hK6 is quantitated in the sample by measuring fluorescence of the fluorescent complexes. The antibodies specific for hK6 may be directly or indirectly labelled with an enzyme. Enzymes are selected based on the ability of a substrate of the enzyme, or a reaction product of the enzyme and substrate, to complex with lanthanide metals such as europium and terbium. Examples of suitable enzymes include alkaline phosphatase and β-galactosidase. Preferably, the enzyme is alkaline phosphatase. The hK6 antibodies may also be indirectly labelled with an enzyme. For example, the antibodies may be conjugated to one partner of a ligand binding pair, and the enzyme may be coupled to the other partner of the ligand binding pair. Representative examples include avidin-biotin, and riboflavin-riboflavin binding protein. Preferably the antibodies are biotinylated, and the enzyme is coupled to streptavidin.

In the method, antibody bound to hK6 in a sample is detected by adding a substrate for the enzyme. The substrate is selected so that in the presence of a lanthanide metal (e.g. europium, terbium, samarium, and dysprosium, preferably europium and terbium), the substrate, or a reaction product of the enzyme and substrate, forms a fluorescent complex with the lanthanide metal. Examples of enzymes and substrates for enzymes that provide such fluorescent complexes are described in U.S. Patent No. 5,3112,922 to Diamandis. By way of example, when the antibody is directly or indirectly labelled with alkaline phosphatase the substrate employed in the method may be 4-methylumbelliferyl phosphate, or 5-fluorosalicyl phosphate. The fluorescence intensity of the complexes is typically measured using a time-resolved fluorometer e.g. a CyberFluor 615 Imunoanalyzer (Nordion International, Kanata, Ontario).

The sample, an antibody specific for hK6, or hK6 may be immobilized. Examples of suitable carriers are agarose, cellulose, dextran, Sephadex, Sepharose, liposomes, carboxymethyl cellulose polystyrene, filter paper, ion-exchange resin, plastic film, plastic tube, glass beads, polyamine-methyl vinylether-maleic acid copolymer, amino acid copolymer, ethylene-maleic acid copolymer, nylon, silk, etc. The carrier may be in the shape of, for example, a tube, test plate, well, beads, disc, sphere etc. The immobilized antibody may be prepared by reacting the material with a suitable insoluble carrier using known chemical or physical methods, for example, cyanogen bromide coupling.

In accordance with an embodiment, the present invention provides means for determining hK6 in a blood sample or tumor tissue extract, preferably a serum sample, by measuring hK6 by immunoassay. It will be evident to a skilled artisan that a variety of immunoassay methods can be used to measure hK6. In general, an hK6 immunoassay method may be competitive or noncompetitive. Competitive methods typically employ an immobilized or immobilizable antibody to hK6 (anti-hK6) and a labeled form of hK6. Sample hK6 and labeled hK6 compete for binding to anti-hK6. After separation of the resulting labeled hK6 that has become bound to anti-hK6 (bound fraction) from that which has remained unbound (unbound fraction), the amount of the label in either bound or unbound fraction is measured and may be correlated with the amount of hK6 in the test sample in any conventional manner, e.g., by comparison to a standard curve.

Preferably a non-competitive method is used for the determination of hK6, with the most common method being the "sandwich" method. In this assay, two anti-hK6 antibodies are employed. One of the anti-hK6 antibodies is directly or indirectly labeled (sometimes referred to as the "detection antibody") and the other is immobilized or immobilizable (sometimes referred to as the "capture antibody"). The capture and detection antibodies can be contacted simultaneously or sequentially with the test sample. Sequential methods can be accomplished by incubating the capture antibody with the sample, and adding the detection antibody at a predetermined time thereafter (sometimes referred to as the "forward" method); or the detection antibody can be incubated with the sample first and then the capture antibody added (sometimes referred to as the "reverse" method). After the necessary incubation(s) have occurred, to complete the assay, the capture antibody is separated from the liquid test mixture, and the label is measured in at least a portion of the separated capture antibody phase or the remainder of the liquid test mixture. Generally it is measured in the capture antibody phase since it comprises hK6 bound by ("sandwiched" between) the capture and detection antibodies.

In a typical two-site immunometric assay for hK6, one or both of the capture and detection antibodies are polyclonal antibodies. The label used in the detection antibody can be selected from any of those known conventionally in the art. The label may be an enzyme or a chemiluminescent moiety, but it can also be a radioactive isotope, a fluorophor, a detectable ligand (e.g., detectable by a secondary binding by a labeled binding partner for the ligand), and the like. Preferably the antibody is labelled with an enzyme which is detected by adding a substrate that is selected so that a reaction product of the enzyme and substrate forms fluorescent complexes. The capture antibody is selected so that it provides a means for being separated from the remainder of the test mixture. Accordingly, the capture antibody can be introduced to the assay in an already immobilized or insoluble form, or can be in a immobilizable form, that is, a form which enables immobilization to be accomplished subsequent to introduction of the capture antibody to the assay. An immobilized capture antibody may comprise an antibody covalently or noncovalently attached to a solid phase such as a magnetic particle, a latex particle, a microtiter plate well, a bead, a cuvette, or other reaction vessel. An example of an immobilizable capture antibody is antibody which has been chemically modified with a ligand moiety, e.g., a hapten biotin, or the like, and which can be subsequently immobilized by contact with an immobilized form of a binding partner for the ligand, e.g., an antibody, avidin, or the like. In an embodiment, the capture antibody may be immobilized using a species specific antibody for the capture antibody that is bound to the solid phase.

A particular sandwich immunoassay method of the invention employs two antibodies reactive against hK6, a second antibody having specificity against an antibody reactive against hK6 labelled with an enzymatic label, and a fluorogenic substrate for the enzyme. In an embodiment, the enzyme is alkaline phosphatase (ALP) and the substrate is 5-fluorosalicyl phosphate. ALP cleaves phosphate out of the fluorogenic substrate, 5-fluorosalicyl phosphate, to produce 5-fluorosalicylic acid (FSA). 5-Fluorosalicylic acid can then form a highly fluorescent ternary complex of the form FSA-Tb(3+)-EDTA, which can be quantified by measuring the Tb3+ fluorescence in a time-resolved mode. Fluorescence intensity is measured using a time-resolved fluorometer as described herein.

The above-described immunoassay methods and formats are intended to be exemplary and are not limiting since, in general, it will be understood that any immunoassay method or format can be used in the present invention.

The methods of the invention can be carried out using a diagnostic kit for quantitating hK6 in a sample. By way of example, the kit may contain antibodies specific for hK6, antibodies against the antibodies labelled with an enzyme; and a substrate for the enzyme. The kit may also contain microtiter plate wells, standards, assay diluent, wash buffer, adhesive plate covers, and/or instructions for carrying out a method of the invention using the kit.

Antibodies specific for hK6 may also be used in imaging methodologies in the management of ovarian cancer. The invention provides a method for imaging tumors associated with hK6 and optionally one or more other kallikreins, preferably kallikreins associated with ovarian cancer, including but not limited to hK4, hK5, bK8, hK9, hK10 and hK11.

The invention also contemplates kits for carrying out the methods of the invention. The kits include an antibody or an antibody fragment which binds specifically to an epitope of a kallikrein, and means for detecting binding of the antibody to its epitope associated with tumor cells, either as concentrates (including lyophilized compositions), which may be further diluted prior to use or at the concentration of use, where the vials may include one or more dosages. Where the kits are intended for in vivo use, single dosages may be provided in sterilized containers, having the desired amount and concentration of agents. Containers that provide a formulation for direct use, usually do not require other reagents, as for example, where the kit contains a radiolabelled antibody preparation for in vivo imaging.

The following non-limiting examples are illustrative of the present invention:

### Example 1

### Immunofluorometric Assay of Human Kallikrein 6 (Zyme/Protease M/Neurosin)Materials and Methods

Diflunisal phosphate (DFP) was synthesized in the laboratory (diflunisal, obtained from Sigma Chemical Co., St. Louis, MO). The stock solution of DFP was 0.01 mol/L in 0.1 mol/L NaOH. DFP stock solutions are stable at 4°C for at least 6 months. Alkaline phosphatase-labeled goat anti-rabbit IgG (GARIg-ALP) and sheep anti-mouse immunoglobulin G (Fc fragment-specific) were obtained from Jackson Immunoresearch, West Grove, PA. Working solutions of GARIg-ALP were prepared by diluting the stock solution 3,000-fold in the assay buffer (described below). White, opaque 12-well polystyrene microtiter strips were obtained from Dynatech Labs., Alexandria, VA. The substrate buffer was a Tris buffer (0.1 mol/L, pH 9.1) containing 0.1 mol of NaCl and 1 mmol of MgCl₂ per liter. The substrate working solution (DFP, 1 mmol/L in substrate buffer) was prepared just before use by diluting the DFP stock solution 10-fold in the substrate buffer. The wash solution was prepared by dissolving 9 g of NaCl and 0.5 g of polyoxyethylenesorbitan monolaurate (Tween 20) in 1 L of a 10 mmol/L Tris buffer, pH 7.40. The developing solution contained 1 mol of Tris base, 0.4 mol of NaOH, 2 mmol of TbCl₃ and 3 mmol of EDTA per liter (no pH adjustment). The assay buffer A was a 50 mmol/L Tris buffer, pH 7.40, containing 60 g of BSA, 0.5 g of sodium azide, 100 mL of normal goat serum, 25 mL of normal mouse serum, 5 g of bovine IgG and 0.5 g of Tween 20 per liter. The assay buffer B was the same as assay buffer A except that mouse serum was omitted.

### CLINICAL SAMPLES

Several clinical samples were used to examine the presence of hK6. These included serum and urine samples from male and female individuals (healthy blood donors), breast cyst fluids obtained by needle aspiration, breast tumor cytosolic extracts, prepared as described previously (*11*), amniotic fluids, milks from lactating women, seminal plasmas, nipple aspirate fluids (NAFs) and cerebrospinal fluids (CSFs). In addition, a panel of human tissue cytosolic extracts, prepared as previously described were tested (Hassapoglidou, S. et al Oncogene 1993, 8:1501-1509*.*). To establish optimal measuring conditions, all samples were tested at various dilutions. The procedures are in accordance with the ethical standards of the Helsinki Declaration of 1975, as revised in 1983.

All tissues and fluid samples were stored at -80°C until use.

### INSTRUMENTATION

A time-resolved fluorometer, the CyberFluor 615 Immunoanalyzer (MDS Nordion, Kanata, ON, Canada) was used to measure Tb³⁺ fluorescence in white microtiter wells. This procedure has been described in detail elsewhere (Christopoulos, TK, et al Anal Chem 1992, 64:342-346*;* Ferguson RA et al, Clin Chem 1996 42: 675-684).

### PROCEDURES

***Production and purification of recombinant hK6 protein.** Human 293 cells transfected with a plasmid* containing the 1.4-kb hK6 cDNA were subjected to selection by growth in G418 (400 mg/L) for three weeks, after which time stable transformants were isolated. One clone generated identifiable amounts of hK6 protein in the culture medium. This cell line was cultured and the tissue culture supernatant was collected and concentrated by using Centricon ultrafiltration devices (Millipore, Waltham, MA 02454). Purification of hK6 from the concentrated cell culture supernatants was achieved by reversed-phase high pressure liquid chromatography (C-8, Aquapore RP-300, 0.45 x 25 cm, Applied Biosystems, Foster City, CA) using a linear gradient of 0.1% trifluoroacetic acid/acetonitrile. Generally, the gradient increased at a rate of 1% acetonitrile per min. Factions containing hK6 were located by SDS-polyacrylamide gel electrophoresis, collected, lyophylized and stored at-20°C (Little SP et al, J. Bil Chem 1997:272:251135-25142).
***Development of polyclonal antibodies against hK6.*** Purified recombinant hK6 protein was used to immunize rabbits and mice using standard procedures (Campbell Am, Production and purification of antibodies. In: Immunoassay. Diamandis EP Christopoulos TK (eds)00. 95-115, Academic Press, San Diego, 1996). The rabbit and mice antisera were used for the development of the immunofluorometric assay without further purification.
***Coating of microtiter plates with sheep anti-mouse immunoglobulin.*** White polystyrene microtiter wells were coated by incubating overnight 500 ng /100 µL per well of the coating antibody diluted in a 50 mmol/L Tris buffer, pH 7.80. The wells were then washed six times with the wash solution and blocked for 1 hour with 200 µL/well of the blocking solution (10 g/L BSA in 50 mmol/L Tris, pH 7.80). After another six washes, the wells were ready to use.
***hK6 calibration.*** hK6 calibrators of 0, 1, 5, 20, 50 and 200 µg/L were prepared by diluting recombinant purified hK6 protein in a 50 mmol/L Tris buffer, pH 7.80, containing 60 g of BSA and 0.5 g of sodium azide per liter.
***hK6** assay.* Calibrators or samples (100 µL) were pipetted into the microtiter wells and 50 µL of the polyclonal mouse anti-hK6 antiserum, diluted 5,000-fold in assay buffer B, were added. The wells were then incubated with shaking at room temperature for 2 hours and washed six times. To each well, was added 100 µL of rabbit anti-hK6 antibody, diluted 1,000-fold in assay buffer A, incubated for 30 min as described above, and then washed six times. To each well, was added 100 µL of a goat anti-rabbit immunoglobulin, conjugated to alkaline phosphatase, diluted 3,000-fold in assay buffer A and incubated for 30 min, as described above. The wells were then washed six times; 100 µL of 1 mmol/L DFP working substrate solution was added, and the wells were incubated for 10 min, as described above. 100 µL of developing solution was added to each well, the wells were mixed by mechanical shaking for 1 min and the fluorescence was measured with the time-resolved fluorometer. The calibration and data reduction were performed automatically by the CyberFluor 615 Immunoanalyzer.
***High performance liquid chromatography, (HPLC):*** Various biological fluids have been fractionated on a gel filtration column, using the procedures described elsewhere (Yu H, Diamandis EP, Clin Chem 1993: 39:2108-2114*;* Diamandis, EP at al Cliln Chem 1997:43:1365-1371*)*)*.* HPLC fractions were collected and analyzed for hK6 with the developed immunofluorometric assay.

### Results

### ASSAY OPTIMIZATION

Two polyclonal antibodies against recombinant hK6 protein were used, one developed in mice and one developed in rabbits. The chosen assay configuration (indirect coating of the wells with a sheep anti-mouse antibody and detection of the immunocomplex with a goat anti-rabbit immunoglobulin, conjugated to alkaline phosphatase) demonstrated good sensitivity (see below) without the need for any purification or conjugation of the primary antibodies. The amounts of antibodies used, the diluents and incubation times of the various assay steps were optimized. Optimal conditions were selected based on the lowest achievable detection limit and best assay linearity and dynamic range. The final conditions are described above.

### CALIBRATION CURVE, DETECTION LIMIT, PRECISION

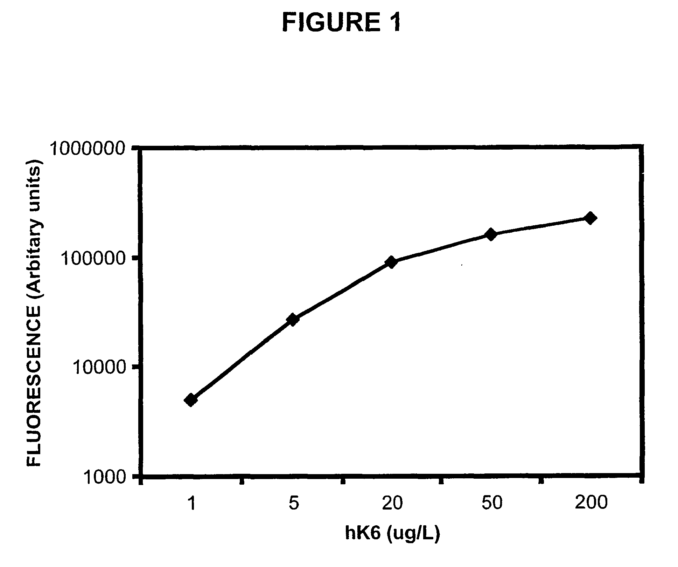

A typical calibration curve of the proposed hK6 assay is shown in Figure 1. The detection limit, defined as the concentration of hK6 corresponding to the fluorescence of the zero calibrator plus two standard deviations, is ≤ 0.5 µg/L. Within-run and between-run precision was assessed at various hK6 concentrations between 2-50 µg/L and with various clinical samples. In all cases, the coefficients of variation (CVs) were between 2 and 9%, consistent with the precision of typical microtiter plate-based immunoassays.

### SPECIFICITY

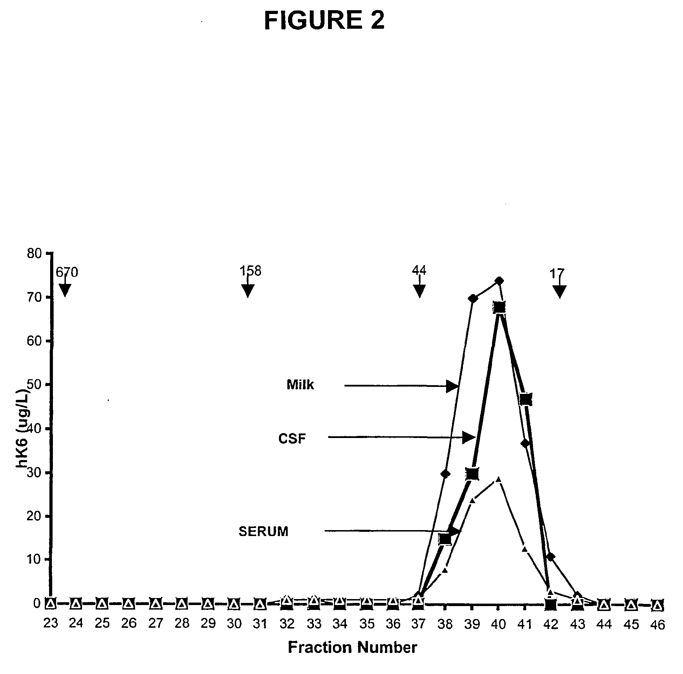

hK6 protein was detected in various biological fluids. In order to ensure that the immunofluorometric assay measures hK6 with high sensitivity and specificity, separated in a gel filtration column three biological fluids with relatively high hK6 concentration, (namely one human milk from a lactating woman, one cerebrospinal fluid and one serum sample from an ovarian cancer patient who was found to have high levels of this biomarker in serum) were separated in and measured on a gel filtration column. The results are shown in Figure 2. In all three biological fluids tested, a single immunoreactive species of a molecular mass of - 30 kDa was detected, which is consistent with the molecular mass of hK6 protein. Higher molecular weight complexes were not detected suggesting that hK6 is present in these biological fluids in its free form. Other serum proteinases (e.g. PSA) are present in serum and other fluids mostly bound to proteinase inhibitors (Stenman U-H, et al, Cancer Res. 1991: 51:222-226); Christensson A et al Cur J Biochem 1990; 194; 755-763).

### hK6 IN BIOLOGICAL FLUIDS AND TISSUE EXTRACTS

To obtain preliminary information on the presence of hK6 in biological fluids, various clinical samples were analyzed, as shown in Table 1. The highest concentration of hK6 was found in milk of lactating women, followed by cerebrospinal fluid, nipple aspirate fluid and breast cyst fluid. hK6 was also detected in male and female serum samples, in the majority of seminal plasmas and in a relatively small percentage of amniotic fluids and breast tumor cytosolic extracts. hK6 protein was not detected in urine.

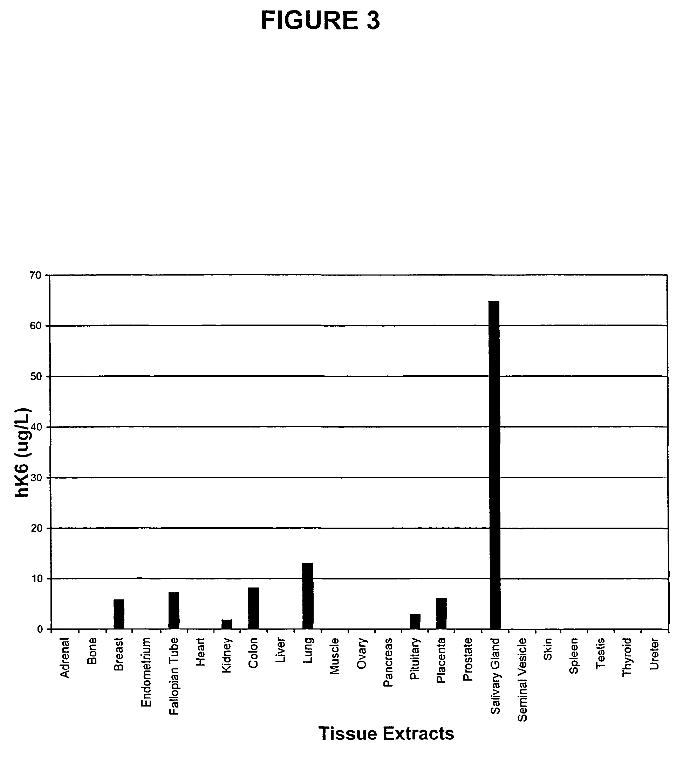

A number of human tissue cytosolic extracts were also tested. The highest concentration of hK6 was detected in the salivary glands, followed by lung, colon, fallopian tube, placenta, breast, pituitary and kidney. The following tissues tested negative: skin, spleen, bone, thyroid, heart, urerter, liver, muscle, endometrium, testis, pancreas, seminal vesicle, ovary, adrenals and prostate (Figure 3).

### Discussion

The present inventors have developed polyclonal antibodies and an immunofluorometric procedure suitable for quantifying hK6 protein in biological fluids and tissue extracts. Since a rich natural source of hK6 protein is not known, recombinant hK6 protein was used for the development of polyclonal rabbit and mice antibodies. This recombinant protein ensures high purity without any contaminating proteins. The chosen assay configuration does not need any further purification or conjugation of the primary antibodies used, and it is thus a convenient method for developing sensitive immunofluorometric procedures. The same principle has been adopted previously for measuring the p53 tumor suppressor in biological fluids (Hassapoglidou S et al, Oncogene 1993: 8: 1501-1509).

The developed immunoassay for hK6 protein demonstrates good sensitivity, dynamic range and linearity (Figure 1). It has been further verified that this assay detects a single immunoreactive band in the biological fluids examined. In serum, this proteinase is present in its free form, similarly to observations with hK2 measurements (Black, MH et al Clin Chem 1999; 45:790-799). However, this is in contrast to the situation with PSA, which is known to be present in serum mainly bound to α₁-antichymotrypsin (Stenman U-H, et al, Cancer Res. 1991: 51:222-226); Christensson A et al Cur J Biochem 1990; 194; 755-763).

The survey of a relatively large number of biological fluids has indicated that hK6 protein is present at relatively high concentrations in milk of lactating women and other breast secretions, including nipple aspirate fluid and breast cyst fluid (Table 1). Previously, the presence of other kallikreins, including PSA and hK2, has been demonstrated in these biological fluids (Yu, H Diamandis; Clin Chem 1995; 41:54-58*;* Sauter ER et al Cancer Epidemiol Biomarkers Prevent 1996 967-970*;* Diamandis Ep et al Breast Cancer Res Treat 199638:259-264*;* Balck MH et al Br J Cancer 2000; 82:361-367*;* Blcak MH et al Clin Chem 1999;45: 790-799*;* yu H. and Diamandis EP Clin Chem 1995:41:204-210*;* Black MH Diamandis EP, Breast Cancer Res Treat 2000 59:1-14*).* Large amounts of hK6 protein were detected in cerebrospinal fluid, which are consistent with the observation that hK6 is expressed at high levels in brain tissue (Little, supra). hK6 was also found in male and female sera and seminal plasmas and in a small percentage of amniotic fluids and breast tumor cytosols. Previously, PSA and hK2 was demonstrated in these biological fluids as well (Yu, H Diamandis; Clin Chem 1995: 41:54-58*;* Sauter ER et al Cancer Epidemiol Biomarkers Prevent 1996 967-970*;* Diamandis Ep et al Breast Cancer Res Treat 199638: 259-264*;* Balck MH et al Br J Cancer 2000; 82:361-367*;* Blcak MH et al Clin Chem 1999;45: 790-799*;* yu H. and Diamandis EP Clin Chem 1995:41:204-210*;* Black MH Diamandis EP, Breast Cancer Res Treat 2000 59:1-14*).* It is interesting to note that although seminal plasma contains extremely high levels of PSA and hK2 (Diamandis EP Trends Endocrinol Metab 1999: 25:14-26' RittenhouseHe et al Crit Rev Clin Lab Sci 1998: 35:275-368), the assay described herein detected very small amounts of hK6 in this biological fluid (Table 1). This further demonstrates that the homologous proteins PSA and hK2 do not have any major cross-reactivity with the developed hK6 assay.

The assay developed here represents the first method for detecting hK6 protein in biological fluids. The results further demonstrate that hK6 is a secreted protein, as predicted by its deduced amino acid sequence (Yousek GM et al Genomics 1999;62:251-259).

### Example 2

### Materials and Methods

### Immunofluorometric assay for hK6

The details of this immunofluorometric assay have been described (See Example 1 and ref. 12). The assay utilizes two hK6-specific polyclonal antibodies, one raised in mouse and the other raised in rabbit. This is a non-competitive immunofluorometric procedure which incorporates the principles of time-resolved fluorometry for detection. The assay measures hK6 in the range of 0.5-200 µg/L with precision < 10%. Serum samples were analyzed without sample pretreatment.

### Clinical samples

For this investigation, leftover serum samples obtained from patients with various malignancies were used (Table 2). Patients were included with relatively high tumor burden (as indicated by tumor marker levels of at least 10-fold higher than the upper limit of normal) in order to increase the chance of detecting possible hK6 elevations in serum. All serum samples were stored at -20°C until analysis for a maximum time of one year. The procedures are in accordance with the Ethical Standards of the Helsinki Declaration of 1975, as revised in 1983.

### Analysis of tumor markers

The tumor markers CA125, PSA, CEA and AFP were analyzed on the Elecsys immunoassay analyzer (Roche Diagnostics, Indianapolis, IN). CA15.3, CA19.9 and hCG were analyzed on the Immuno 1 immunoassay analyzer (Bayer Diagnostics, Tarrytown, NY) and calcitonin was measured with a radioimmunoassay kit from Diasorin, Italy. The upper limit of normal values for the tumor markers were 35 KU/L (CA125), 4 µg/L (PSA), 10 µg/L (AFP), 5 µg/L (CEA), 35 KU/L (CA15.3), 37 KU/L (CA19.9), 10 IU/L (hCG) and 100 ng/L (calcitonin).

### Results

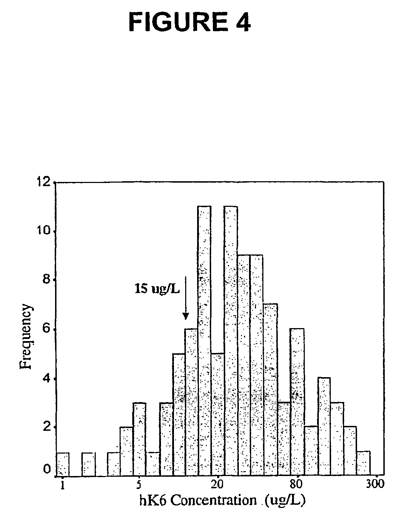

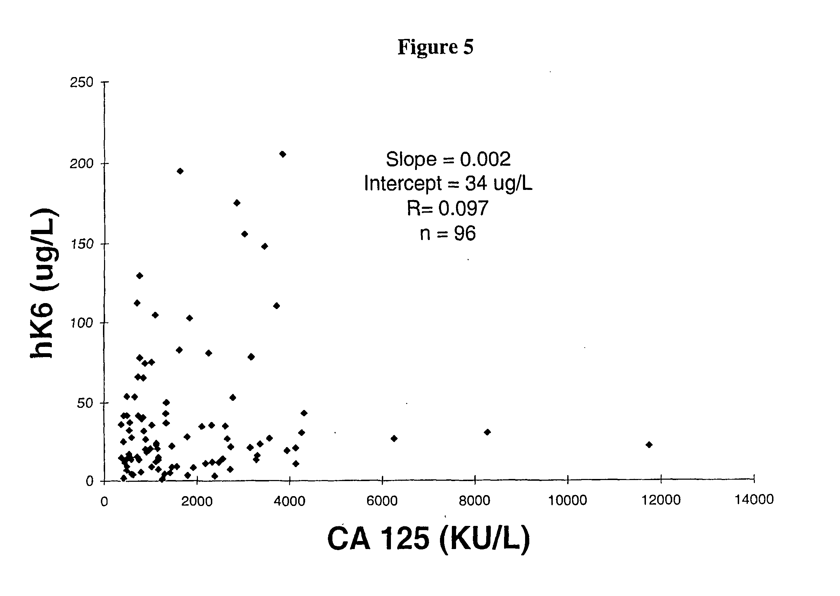

A total of 378 serum samples were analyzed with the previously described immunofluorometric assay for hK6 (12). These samples were from either normal individuals (male and female) or from patients with various malignancies. The obtained data are shown in Table 2. While in none of the normal controls and in only two samples from patients with non-ovarian malignancies the hK6 concentration was above 15 µg/L (at arbitrary cutoff), the majority of patients with ovarian carcinoma (~ 66%) had highly elevated hK6 concentrations in their serum (>15 µg/L). The distribution of hK6 values in serum of ovarian cancer patients is shown in Figure 4. As shown in Figure 5, the correlation between hK6 concentrations and CA125 levels is poor and not statistically significant.

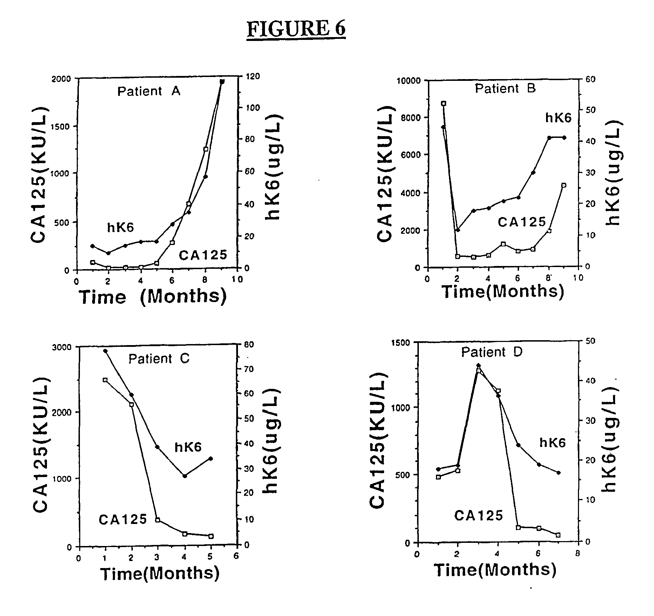

In Figure 6, data is presented on temporal changes of serial serum hK6 and CA125 concentration in four patients with ovarian cancer. The hK6 concentration changes during the monitoring period, similarly to CA125, suggesting that this new biomarker may have value for patient management.

### Discussion

The data of Table 2 summarize the findings and demonstrate that among all cancer types tested (normal males and females versus breast, thyroid, testicular, gastrointestinal, prostate, lung and ovarian cancer), only ovarian cancer patients show significantly elevated levels of this biomarker in the circulation. Approximately 66% of patients had levels higher than 15 µg/L, a cutoff that affords 98-100% specificity for all other cancers tested. Although these data are highly promising, regarding value of hK6 as a circulating biomarker for ovarian carcinoma, it should be taken into consideration that all patients with ovarian cancer had relatively high levels of CA125 (≥ 372 KU/L, which is approximately 10 times higher than the upper reference range). The data of Figure 6 indicate that serum levels of hK6 change with time during ovarian cancer monitoring, suggesting that this biomarker may be useful for monitoring patients after primary treatment.

As is evident from Figure 5, there is no significant correlation between hK6 concentration and CA125, suggesting that these two biomarkers may be complementary for the diagnosis and management of ovarian carcinoma.

In conclusion, the first evidence that serum hK6 concentration is significantly increased in about 66% of ovarian cancer patient is provided. The test seems to be specific for ovarian cancer since no such increases were seen in various other malignancies. Therefore, hK6 represents a novel serum biomarker for ovarian cancer which may be useful for disease diagnosis and monitoring.

### Example 3

### Materials and Methods

### Patient population

Included in this study were 97 apparently healthy women (ages 26 to 72 years; mean = 52, median = 49 years), 141 women with benign disease (ages 21 to 76 years; mean = 46, median = 45 years) and 146 patients with histologically proven primary ovarian carcinoma (ages 28 to 78 years; mean = 56, median = 57 years). Of the benign lesions, 50 were classified as endometriosis, 22 as mucinosum, 10 as ovarian teratomas, 26 as dermoidea, 15 as corpus luteum and 18 as serosum. Tumors were staged according to the International Federation of Gynecology and Obstetrics (FIGO) criteria. Histologic classification was based on the World Health Organization and FIGO recommendations. The characteristics of the ovarian cancer patients in terms of stage, grade, histotype, residual tumor post-surgery, debulking success and response to chemotherapy are shown in Table 7. Serum samples from all patients were collected pre-surgically, before initiation of therapy, and stored at -80°C until analysis. For 105 ovarian cancer patients, serum was also available post-surgery. This sample was obtained approximately 2-3 weeks post-surgery.

Sera were obtained from four centres as follows: The Gynecologic Oncology Unit, University of Turin, Italy (97 normals, 14 benign, 21 cancers); Holland (40 cancers); Belgium (13 benign, 85 cancers); Department of Clinical Chemistry, Helsinki University Central Hospital, Finland (114 benign).

Patients were monitored for survival and disease progression for a median duration of 25 months (range 1-106 months). Follow-up information was available for 131 of the ovarian cancer patients. Sixty-four (49%) of these relapsed and 28 (21 %) died during the course of the follow-up period.

### Analysis of hK6 and CA125

CA125 was measured with a commercially available automated immunoassay method (Immulite 2000, Diagnostic Products Corporation, Los Angeles, CA). The upper limit of normal for this method is 23 KU/L. The concentration of hK6 was measured with a procedure described herein (12) with some modifications. This assay employs a monoclonal anti-hK6 mouse antibody, coated directly on microtiter wells (capture antibody), a polyclonal rabbit detection antibody and an alkaline phosphatase-conjugated goat anti-rabbit antibody. Signal was quantified by time-resolved fluorometry. The assay has a detection limit of 0.1 µg/L and a dynamic range up to 50 µg/L. Precision was <10% within the measurement range. The serum samples were analyzed in duplicate with inclusion of three quality control samples in every run.

### Statistical analysis

To analyze data, patients were divided into different groups according to clinical and pathological parameters. The analyses of differences between hK6 serum concentration before and after surgery were performed with the non-parametric McNemar test. The binomial distribution was used to compute the significance level of the McNemar test.

Receiver operating characteristic (ROC) curves were constructed for hK6 and CA125 serum concentration by plotting sensitivity versus (1-specificity) and the areas under the ROC curves (AUC) were calculated. The non-cancer group included the normal individuals and the patients with benign disease. Correlations between different variables were assessed by the Spearman correlation coefficient. The non-parametric Mann-Whitney U test was used to determine differences between two groups and the non-parametric Kruskal-Wallis test was used for the analysis of differences among more than two groups. These tests treated hK6 concentration in serum as a continuous variable. hK6 serum concentration was also classified as either hK6-positive (> 4.4 µg/L) or hK6-negative (≤ 4.4 µg/L). The relationship of this dichotomous variable with other clinicopathological correlates was established with the Chi Square (χ²) test or the Fisher's Exact test, as appropriate.

Kaplan-Meier progression-free survival and overall survival curves were constructed to demonstrate the survival differences between the hK6-positive and hK6-negative patients. The log rank test was used to examine the significance of the differences among the survival curves. The impact of serum hK6 concentration on patient overall survival (OS) and on progression of the disease (progression-free survival; PFS) was assessed with the hazards ratio, calculated by both univariate and multivariate Cox proportional hazards regression models. In the multivariate analysis, the clinical and pathological variables that may affect survival, including stage of disease, tumor grade, residual tumor and histologic type were adjusted.

### Results

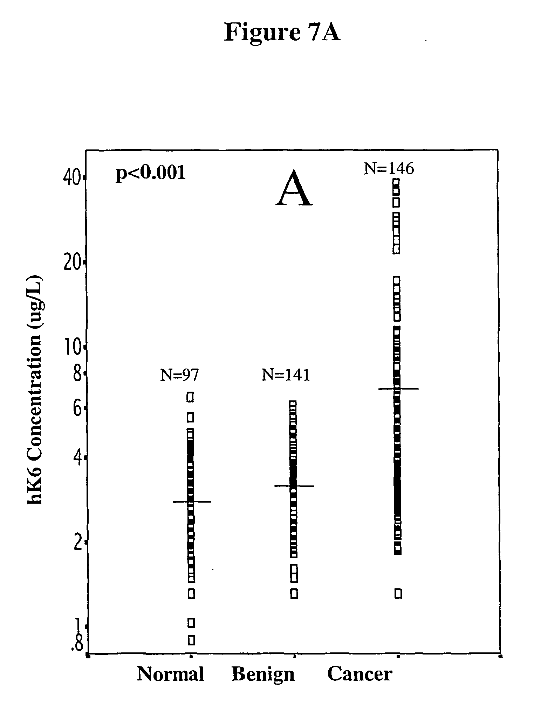

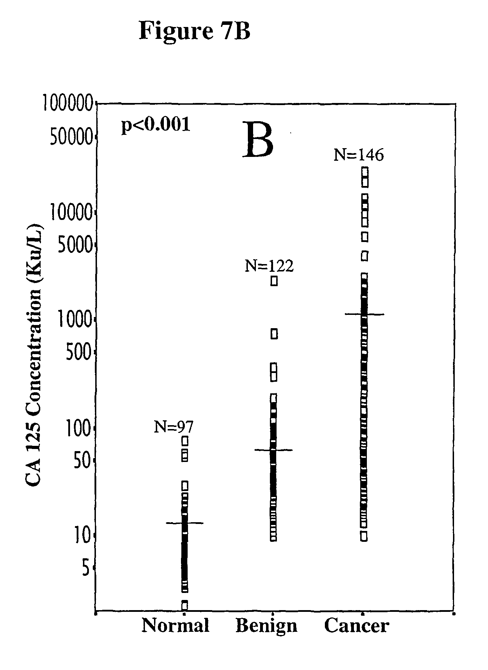

Serum hK6 concentration in cancer and non-cancer patients: The mean, median, range and selected percentiles of serum hK6 concentration among non-cancer (normal; n = 97), benign disease (n = 141), pre-surgical (n = 146) and post-surgical (n = 105) ovarian cancer patients is shown in Table 3. The mean and median values between non-cancer (normal) and benign disease patients were not statistically significant. The mean and median hK6 values in pre-surgical ovarian cancer patients were significantly higher than the non-cancer and benign groups (p < 0.001). The distribution of hK6 concentration in the three groups of patients (normal, benign, pre-surgical ovarian cancer) is further presented in Figure 7 along with the corresponding CA125 values. Clearly, pre-surgical serum hK6 concentration is not different between normal and benign disease patients but is significantly elevated in a proportion of ovarian cancer patients (Figure 7A). Conversely, CA125 values are progressively increased from normal, to benign, to cancer patients (Figure 7B).

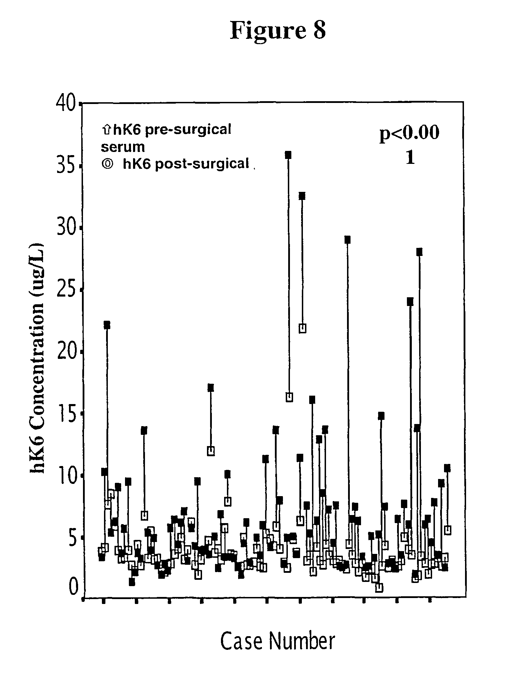

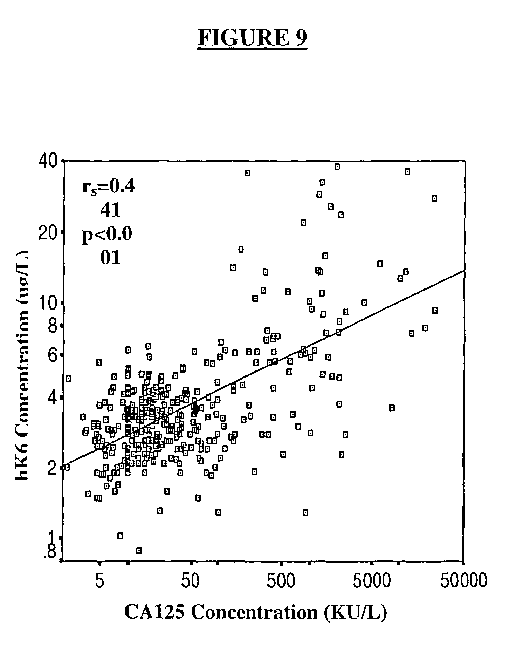

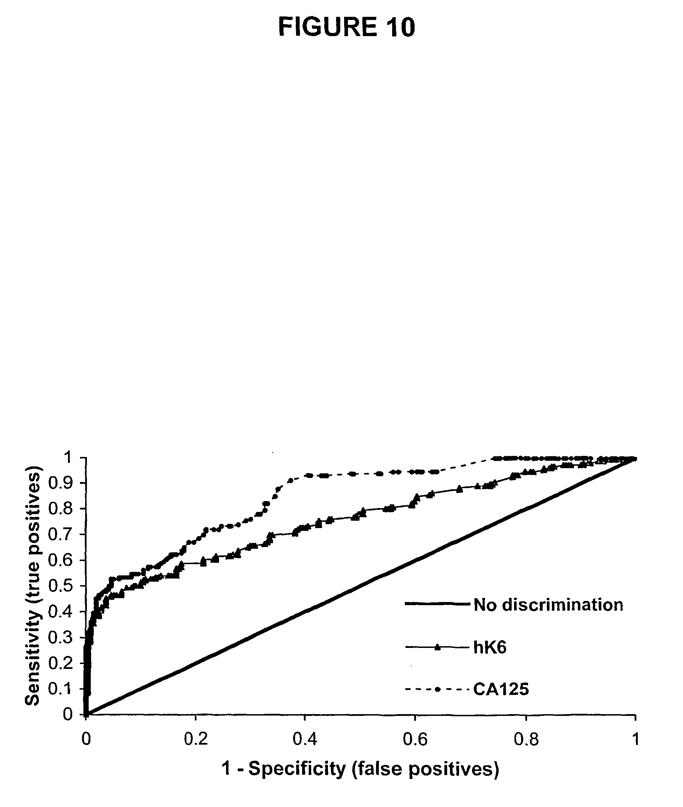

For dichotomous classification of this patient population as hK6-positive and hK6-negative, the hK6 cutoffs of 4.2 µg/L (90% diagnostic specificity) and 4.4 µg/L (95% diagnostic specificity) were selected.
Changes of serum hK6 concentration post-surgery: For 105 patients with ovarian cancer, pre-surgical and post-surgical serum samples were selected. As shown in Figure 8, 71 patients (68%) demonstrated a drop in hK6 concentration post-surgery, 21 (20%) had unchanged values and 13 (12%) had higher hK6 serum levels after the operation. By the McNemar test, the concentration drop post-surgery was statistically highly significant (p < 0.001).
Correlation between serum hK6 and CA125 concentration: The logarithmic plot of Figure 9 shows that there is a weak correlation between serum hK6 and CA125 concentration (Spearman correlation rₛ = 0.44). While the correlation is significant, there are still many samples with quite variable values. For example, at CA125 levels around 500 KU/L, hK6 concentration ranges from 2-40 µg/L while samples with hK6 levels around 6 µg/L may have CA125 values ranging from 5 to > 5,000 KU/L.
Diagnostic sensitivity and specificity of serum hK6 concentration: For this calculation, the various subgroups of patients were considered, as shown in Table 4. In the non-cancer group, all patients who are either normal or have benign disease were included. When the whole patient group was analyzed, diagnostic sensitivity is around 54% at 90% specificity and 50% at 95% specificity. The receiver operating characteristic (ROC) curve of Figure 10 indicates a slight diagnostic advantage of CA125, in comparison to hK6. However, the two markers can work in combination, since hK6 concentration could be elevated in a subset of patients with relatively low CA125. In the subgroup of patients with CA125 > 60 KU/L, the diagnostic sensitivity of hK6 is 71 and 65% at specificities of 90 and 95%, respectively. In the subgroup of patients with low CA125 (< 23 KU/L), about 13-17% of patients will still have elevated hK6, at hK6 cut-offs of 4.4 (95% specificity) or 4.3 µg/L (90% specificity), respectively. In the subgroup of patients with slightly elevated CA125 (23-60 KU/L), the diagnostic sensitivity of hK6 is 15-26% at specificities of 95-90%, respectively (Table 4).

In Table 5, the additional contribution of hK6 in identifying ovarian cancer patients was calculated by using either CA125 alone or CA125 plus hK6. Among all patients with known stage (N = 124), hK6 analysis increases the sensitivity of CA125 by 12% or 13%, at 90% or 95% specificity cut-offs for both markers. The contribution is still significant at ovarian cancer stages I/II (43 patients). The addition of hK6 increases the sensitivity of CA125 alone from 30% to 42%, or from 26% to 37%, at 90% or 95% specificity cut-offs for both markers, respectively.

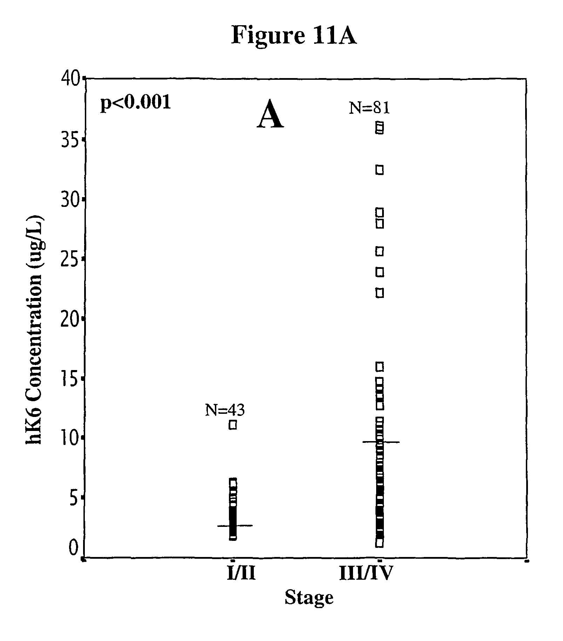

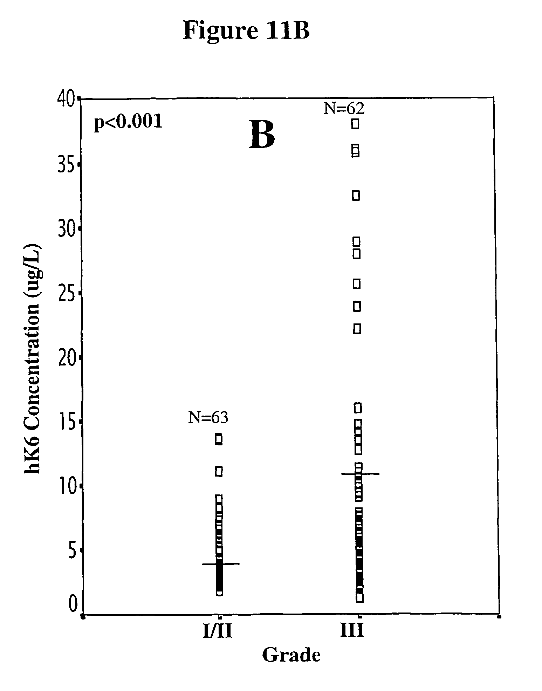

Table 6 summarizes the relative risk (RR) of having ovarian cancer, based on serum hK6 concentration. The relative risk increases exponentially with increasing hK6 concentration, reaching a value of 20 when hK6 is ≥ 4.3 µg/L. The RR is still substantial (RR = 5.3) in multivariate analysis, after adjusting for CA125 levels.
Prognostic value of serum hK6: Higher ovarian cancer stage and grade are strongly associated with higher serum hK6 concentration (Figure 11 and Table 7). Furthermore, serous adenocarcinomas are more frequently associated with high serum hK6 concentration (positivity 68%) followed by endometrioid tumors (positivity 33%); mucinous tumors are rarely associated with high serum hK6 (9%). Furthermore, high serum hK6 concentration is associated with presence of residual tumor, suboptimal debulking and poor response to chemotherapy. All these associations were highly significant (p < 0.001).

In univariate Cox analysis, serum hK6 concentration is associated with shorter progression-free and overall survival (Table 8). These associations remained statistically significant in the multivariate analysis. The prognostic value of CA125 was no longer statistically significant in the multivariate analysis. Besides pre-surgical serum hK6, stage of disease was the only other parameter that was associated with both progression-free and overall survival in multivariate analysis (Table 6).

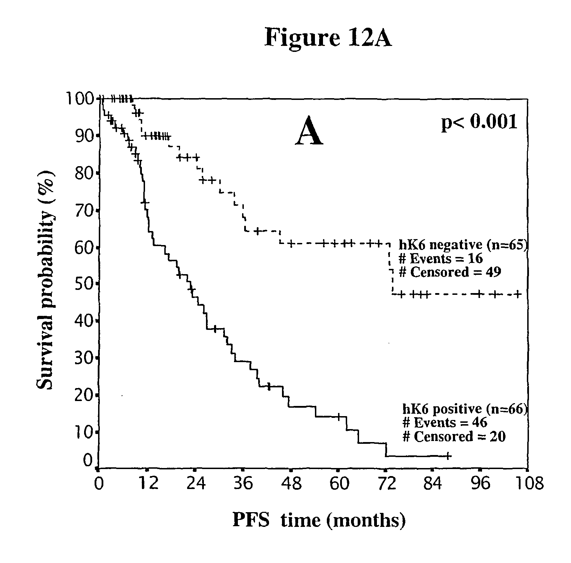

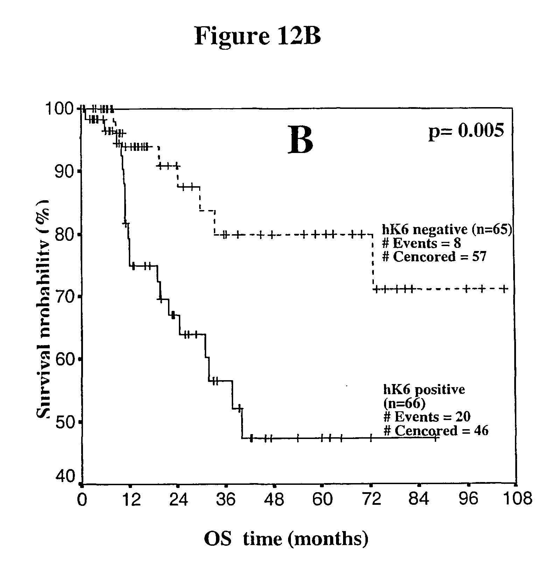

Similar data were obtained with Kaplan-Meier survival analysis (Figure 12). Patients with high pre-surgical serum hK6 have much shorter progression-free and overall survival than patients with low pre-operative hK6 levels. While virtually all patients with high serum hK6 relapsed by 6 years, more than 50% of patients with low pre-operative serum hK6 were still in remission.

### Discussion

The discovery of new ovarian cancer biomarkers for early diagnosis, prognosis, monitoring and prediction of therapeutic response will likely contribute to improved clinical outcomes. The only well accepted ovarian cancer biomarker, CA125, was discovered 20 years ago. A number of other potential ovarian cancer biomarkers have been identified but their clinical value is not established (27). A novel ovarian cancer biomarker, human kallikrein 6 (hK6), a member of the expanded human kallikrein gene family, is described herein.

The traditional ovarian cancer biomarker, CA125, falls short of being able to diagnose early ovarian cancer. In addition to its low sensitivity for early disease, CA125 also suffers from low specificity i.e. elevated levels are seen in many benign abdominal diseases. Currently, it is widely accepted that no single cancer biomarker will provide all the necessary information for optimal cancer diagnosis and management. The current trend is to focus on the identification of multiple biomarkers which can be used in combination. Such approaches have already shown to have clinical potential in ovarian cancer (28,29).

Serum hK6 represents a novel biomarker for ovarian carcinoma. This biomarker is more specific for ovarian cancer than CA125 since, in contrast to CA125, elevations were not seen in benign diseases (Figure 7). The diagnostic sensitivity of hK6 is slightly less than the diagnostic sensitivity of CA125 at the same specificity cut-offs (Table 5 and Figure 10). However, hK6 can increase the diagnostic sensitivity of CA125 at all stages of the disease, including stage I/II disease (Table 5). Despite the weak correlation between hK6 and CA125 (Figure 9), there are still patients with normal CA125 who have elevated hK6 levels (Table 4). Thus, CA125 and hK6 could be used in combination to increase the diagnostic sensitivity of each of the biomarkers alone.

Similarly to the situation with CA125, hK6 concentration is more frequently elevated in serous ovarian carcinoma than in endometrioid and mucinous carcinomas (Table 7). Serum hK6 concentration is also more frequently elevated in late stage and higher grade disease. Serum hK6 concentration is a powerful predictor of patient outcomes. Patients with pre-operative hK6 concentration above 4.4 µg/L have significantly worse prognosis than patients with low pre-operative hK6 (Table 8 and Figure 12). Serum hK6 concentration is a more powerful prognostic indicator that serum CA125. The prognostic value of CA125 disappears in multivariate analysis while serum hK6 is an independent prognostic indicator, as shown in the multivariate analysis of Table 8. Serum hK6 likely originates from tumor cells, since post-operatively, the levels are significantly decreased (Figure 8). In the study in Example 4 examining the prognostic value of hK6 analysis in ovarian tumor extracts, the overexpression of hK6 in tumor cells was verified by immunohistochemistry and further provided evidence that intratumor hK6 concentration is also a strong predictor of prognosis. Interestingly, many other members of the human kallikrein gene family, including the enzymes hK4, hK5, hK7, hK8, hK9 and hK10 have already shown to have prognostic significance in ovarian cancer (34-41). Serine proteases not belonging to the kallikrein family have also been shown to have prognostic significance in ovarian cancer, including trypsin, hepsin and testisin (42-44). Yet, it has been known for years that many other proteolytic enzymes have prognostic value in many cancers (for reviews, see 45 and 46). The biological mechanisms of proteolytic enzyme involvement in cancer prognosis includes their ability to degrade extracellular matrix, thus facilitating invasion and metastasis (47-49). It seems likely that multiple members of the human kallikrein gene family are disregulated in ovarian cancer. It is thus possible that other members of this protease family may emerge as potential ovarian cancer biomarkers. If these proteases are involved in cancer progression, they may be suitable candidates as therapeutic targets.

Table 7 shows preliminarily that pre-surgical serum hK6 concentration may be a predictor of response to chemotherapy in ovarian cancer patients. Among the non-responders, 81 % had elevated pre-surgical hK6 concentration while 19% of these patients had low hK6 concentration. Among the patients who had either complete or partial response to chemotherapy, 57% had low pre-operative hK6 concentration (p < 0.001).

In conclusion, serum hK6 concentration represents a novel biomarker for ovarian carcinoma, which has potential utility as a diagnostic, prognostic and predictive tool. The combination of hK6 and CA125 improves the diagnostic sensitivity of ovarian cancer at all stages, including early stage disease.

### Example 4

### KLK6 Ovarian Tissue

### PATIENTS AND METHODS

**Ovarian Cancer Patients.** One hundred eighty patients with primary ovarian cancer were included in this study. These patients underwent surgery for ovarian cancer at the Department of Gynecology, University of Turin, Italy. Patient age ranged from 25 to 82 years with a median of 59 years. Clinical and pathological information documented at the time of surgery included clinical stage of the cancer, grade and histology of the tumor, and amount of remaining tumor. Menopausal status was documented and response to chemotherapy monitored. Tumors were staged according to the International Federation of Gynaecology and Obstetrics (FIGO) criteria. Histologic classification was based on the World Health Organization and FIGO recommendations. Of the tumors included in this study, 80 were classified as serous papillary, 32 as undifferentiated, 27 as endometrioid, 13 as mucinous, 14 as clear cell, 10 as mullerian and 4 as other. The size of the residual tumors ranged from 0 to 9 cm, with a median of 1.1 cm.

Patients were monitored for survival and disease progression (no apparent progression or progression) for a median duration of 62 months (range 1-99 months). Follow-up information was available for 165 of the patients. 97 (54%) of these relapsed and 61 (34%) died during the course of the follow-up period.

Investigations were carried out in accordance with the ethical standards of the Helsinki Declaration of 1975, as revised in 1983, and were approved by the Institute of Obstetrics and Gynecology, Turin, Italy.
**Preparation of Tumor Cell Extracts.** Tumor tissue was frozen in liquid nitrogen immediately after surgery and stored at -80°C until extraction. 20 to 100 mg of frozen tissue was pulverized on dry ice to a fine powder and added to 10 volumes of extraction buffer (50 mM Tris, pH 8.0, 150 mM NaCl, 5 mM EDTA, 10g/L of NP-40 surfactant, 1 mM phenylmethyl sulphonyl fluoride, 1g/L of aprotinin, 1g/L of leupeptin). The resulting suspension was incubated on ice for 30 minutes during which time it was vortexed every ten minutes. The mixture was then centrifuged at 14,000 rpm at 4°C for 30 minutes and the supernatant (cell extract) was collected and stored at -80°C until analysis. Protein concentration of the extract was determined with the bicinchoninic acid method, with albumin as standard (Pierce Chemical Co., Rockford, IL).
**Measurement of hK6 in Ovarian Cell Extracts.** The concentration of hK6 in tumor cell extract was quantified with a highly sensitive and specific non-competitive immunoassay for hK6 that has been previously described and evaluated in detail (12). The assay incorporated two hK6-specific polyclonal antibodies, one raised in mouse and the other in rabbit, in a sequential two site immunometric format with time resolved fluorescence detection. Analysis of standards, tumor cell extracts and control pools was carried out in duplicate in 96-well polystyrene microtiter plates with 200 µL of specimen added to the immunoassay. The standard curve using recombinant hK6 protein ranged from 0.5 µg/L to 200 µg/L. Assay precision was better than 10%. Signal detection and data reduction were performed automatically by the CyberFluor 615 Immunoanalyzer.
**Localization of hK6 in Ovarian Tumor Specimens by Immunohistochemistry**. A rabbit polyclonal antibody was raised against hK6 full-size recombinant protein, produced in yeast cells. Immunohistochemical staining for hK6 was performed according to a standard immunoperoxidase method. Briefly, paraffin-embedded tissue sections (4 µm) were fixed and dewaxed. Endogenous peroxidase activity was blocked with 3% aqueous hydrogen peroxide for 15 minutes. Sections were then treated with 0.4% pepsin at pH 2.0 for 5 minutes at 42°C and blocked with 20% protein blocker (Signet Labs) for 10 minutes. The primary antibody was then added at 1:400 dilution for 1 hour at room temperature. After washing, biotinylated anti-rabbit antibody (Signet) was added, diluted 4-fold in antibody dilution buffer (DAKO). Following incubation and washing, streptavidin tagged horseradish peroxidase was added for 30 minutes at room temperature. After washing, detection was achieved with amino ethyl carbazole (AEC) for 5-10 minutes. The slides were counterstained with hematoxylin and then mounted with cover slips.
**Statistical Analysis.** Statistical analysis was performed with SPSS software (SPSS Inc. Richmond, CA). To analyze data, patients were divided into different groups according to clinical and pathological parameters. Because the distribution of hK6 mass per mg total protein (i.e. specific activity) in the ovarian tumor extracts was not Gaussian, the non-parametric Mann-Whitney U test was used to determine differences between two groups and the non-parametric Kruskal-Wallis test was used for the analysis of differences among more than two groups. These tests treated hK6 specific activity in the tumor extract (ng hK6/mg total protein) as a continuous variable. hK6 tumor extract specific activity was also classified as either hK6-positive (> 35 ng/mg total protein; see Figure 7B for explanation) or hK6-negative (≤ 35 ng/mg total protein). The relationship of this dichotomous variable to other clinicopathological correlates was established with the Chi Square (χ²) test or the Fisher's Exact Test, as appropriate. The impact of tumor extract hK6 specific activity on patient survival and on progression of the disease (progression-free survival) was assessed with the hazards ratio calculated by both univariate and multivariate Cox proportional hazards regression models (30). In the multivariate analysis, the clinical and pathological variables that may affect survival, including stage of disease, tumor grade, residual tumor, histologic type and age were adjusted. Kaplan-Meier progression-free survival and overall survival curves (31) were constructed to demonstrate the survival differences between the hK6-positive and hK6-negative patients. The log rank test (32) was used to examine the significance of the differences among the survival curves. Following analysis of the entire patient data set as a whole, the process was repeated on subgroups stratified separately by disease stage, by tumor grade and by amount of tumor remaining following surgery (debulking success). The impact of tumor hK6 level (positive or negative) on survival and on disease progression was determined by univariate and multivariate models for each of the subgroups.

### RESULTS

**Distribution of hK6 Specific Activity in Ovarian Tumor Extracts.** The distribution of hK6 specific activity in ovarian tumor extracts from the 180 patients (Figure 13A) ranged from 0.04 ng/mg total protein to 497 ng/mg of total protein with a mean of 33 ng/mg total protein and a median of 13.2 ng/mg total protein. A value of 35 ng/mg total protein was identified by Chi square analysis (χ² = 7.3; P = 0.007) as the optimal cutpoint to distinguish positive from negative tumors in terms of predicting overall survival (Figure 13B). Thirty percent of the tumors were hK6 positive by this criterion. hK6 specific activity in tumor extracts was treated both as a continuous variable and as a dichotomous variable (≤ 35 ng/mg total protein, > 35 ng/mg total protein) in the analyses that follow.

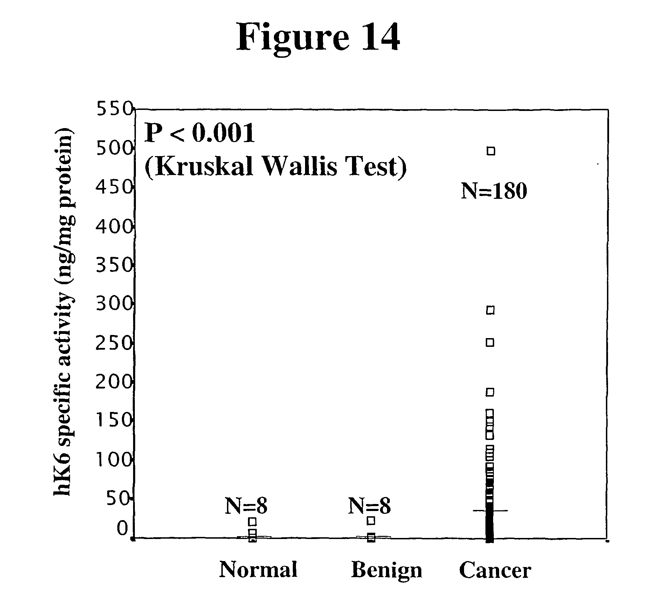

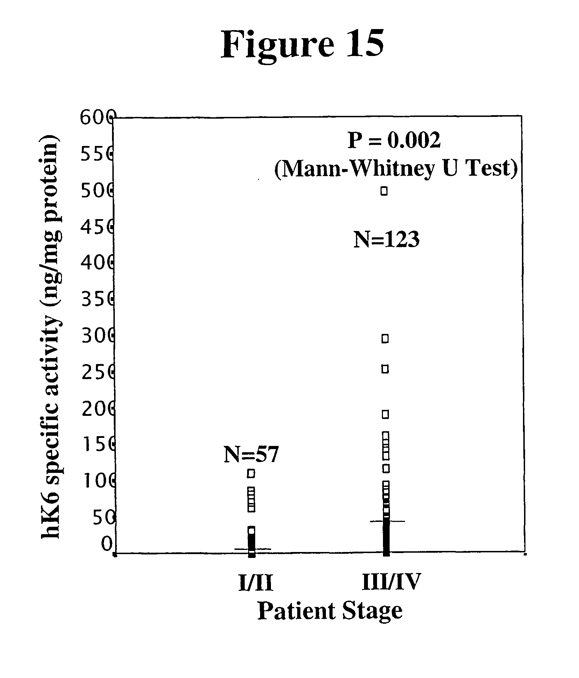

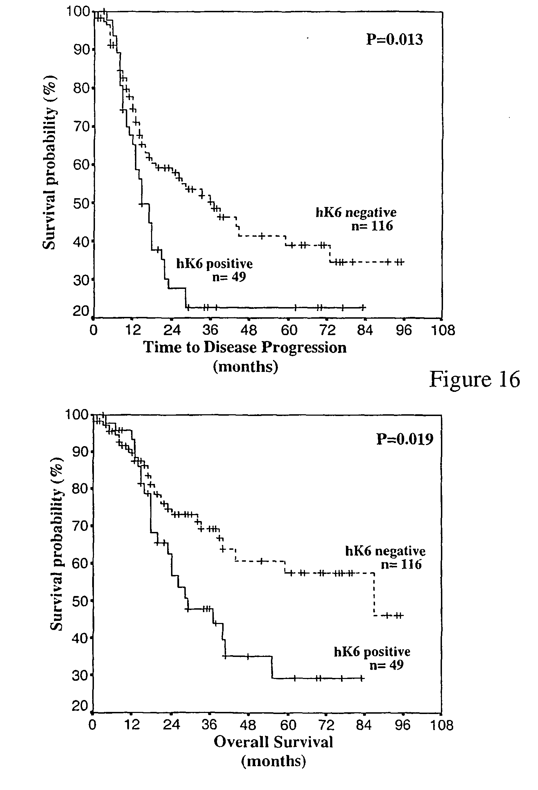

hK6 specific activity (ng hK6/mg total protein) was significantly elevated (P < 0.001 by the Kruskal Wallis test) in extracts of ovarian tumor (mean 32.7, standard error 3.8, range 0.04 to 497) compared to extracts prepared from normal ovarian tissues (mean 3.5, standard error 2.5, range 0.05 to 20.8) or from ovarian tissue with benign disease (mean 3.2, standard error 2.6, range 0.03 to 21.5) (Figure 14). Further analysis showed there was no significant difference in hK6 specific activity among the ovarian tumors when they were stratified by histotype (i.e. serous vs undifferentiated vs endometrioid, etc) (data not shown).
**Relationships between hK6 Status and Other Clinicopathological Variables.** The distributions of various clinicopathological variables between hK6-positive and hK6-negative patients are summarized in Table 9. The relationships between hK6 status and these variables were examined with either the χ² Test or Fisher's Exact Test, as appropriate. No relationship was observed between hK6 status and tumor grade, menopausal status and response to chemotherapy. However, hK6-positive patients were more likely to have advanced disease (stage II-IV), serous tumor histology and greater residual tumor (>1 cm) (all P<0.05). hK6 tumor extract specific activity when treated as a continuous variable also associated proportionally with stage of the disease. Figure 15 shows the distribution of hK6 specific activity stratified by disease stage. hK6 specific activity was significantly higher in extracts from stage III/IV ovarian cancer than in those from stage I/II (P = 0.002 by the Mann Whitney U Test).
**Univariate and Multivariate Survival Analysis.** The impact of hK6 specific activity, other clinicopathological variables and age on disease progression and on overall survival is presented in Table 10. In univariate analysis, hK6-positive patients had a significantly increased risk of disease progression (hazard ratio =1.71) and death (hazard ratio =1.88) (P<0.05). When hK6 specific activity was treated as a continuous variable, hazard ratios were closely similar to those of hK6 negative tumors (arbitrarily set at 1.00), although the slight increase in risk of disease progression (hazard ratio = 1.005) was highly significant at P = 0.001. Kaplan-Meier survival curves demonstrated survival differences between hK6-positive and hK6-negative patients. As Figure 16 shows, the probability of progression-free and overall survival, respectively, are lower in hK6-positive patients than in hK6-negative patients.

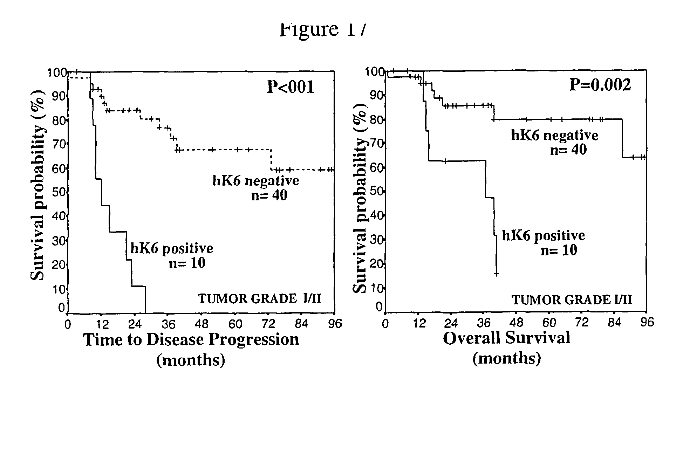

The adverse effects of hK6 positivity on progression free survival and on overall survival were lost in multivariate analysis. As shown in Table 10, when survival outcomes were adjusted for other clinicopathological variables, hK6-positive and hK6-negative patients had statistically similar rates of disease progression and overall survival. Tumor grade also lost its univariate prognostic significance in multivariate analysis. Only stage of disease and residual tumor remaining after surgery maintained their independent effects on survival outcome in the multivariate analysis.
**Univariate and Multivariate Survival Analysis in Subgroups of Patients.** The patients were divided into different subgroups based on disease stage, tumor grade, and debulking success (residual tumor). In each subgroup, the impact of hK6 positivity and negativity on disease progression and on overall survival was determined by univariate and by multivariate Cox proportional hazard regression models. The results are shown in Table 11. hK6 specific activity (positive, negative) significantly impacted survival in the subgroup of patients with tumor grade I or II. Univariate analysis revealed that hK6-positive patients were about 9-times more likely to suffer disease progression and 5-times more likely to die than hK6-negative patients. These survival differences remained significant even after the data were subjected to multivariate analysis. The relative risk of both outcomes arising from hK6 positivity was now about 4-fold (P < 0.03). hK6 status had no such effect among patients with Grade III tumor, nor could any discernible effect be demonstrated among patients with early stage disease and among those with greater than 1 cm of tumor remaining following surgery. Univariate analysis revealed a 2-fold increase in risk of disease progression and of death in the subgroup of patients with advanced disease (stage III and IV) who were hK6 positive, but the effect was lost in the multivariate analysis. The opposite occurred in the subset of patients characterized by optimal debulking of the tumor at the time of surgery (remaining tumor less than 1 cm in diameter). hK6 positivity had no demonstrable adverse effect on disease progression or on survival by univariate analysis, but did become statistically significant, giving a 3.5 and 5.5-fold increase in adverse risk, respectively, when the data were subjected to multivariate analysis. The emergence of effects in the multivariate model when none are generated by the univariate model happens when the adjusted variables have no impact at all on the outcome. In the case here, this means that stage of disease, tumor grade, tumor histology and patient age had no prognostic potential on disease progression and overall survival in this particular subset of patients. Kaplan-Meier survival curves of the subset of patients with grade I or II ovarian tumor are shown in Figure 17. As expected from the univariate analysis mentioned earlier, there was a significant difference in disease progression and survival between hK6 positive and hK6 negative patients.
**Immunohistochemical Staining of hK6 in Ovarian Tumors**. Immunohistochemical staining of hK6 in paraffin embedded tumor sections was roughly proportional to hK6 specific activity in tumor extracts (data not shown). The immunohistochemical localization of hK6 protein in four ovarian tissues that contined benign, borderline or malignant tumor is depicted in Figure 18. hK6 staining was restricted to epithelial cells, being absent in mesenchymal elements including fibrous supporting stroma. hK6 stained within the cytoplasm of epithelial cells, but staining intensity was variable among and within tumor preparations.

### DISCUSSION

Increased hK6 synthesis was found to be predictive of more aggressive tumor behavior over time. Considered apart from other clinicopathological variables and age, hK6 positivity across the entire patient population under study was associated with about a 2-fold increase in the risk of both disease progression and of death. This effect was lost when outcomes were adjusted for the other clinicopathological variables and age in multivariate analysis of the entire patient population, but not when the multivariate analysis was restricted to those patients with lower grade tumor and with less residual tumor remaining after surgery (<1 cm in diameter). Among the former subgroup of patients, hK6 positivity predicted about a 4-fold increase in the risk of disease progression and of death (P < 0.03) while corresponding hazard ratios in the latter subgroup were 3.75 and 5.5, respectively (P < 0.02). The data show that hK6 positivity has independent predictive potential in these two subgroups and gives insight into tumor behavior over time that cannot be gleaned from the clinical parameters and pathological correlates conventionally measured. Hence hK6 testing could contribute to more individualized effective treatment of such patients.

hK6 was found to be frequently overexpressed in ovarian tumors compared to nonmalignant ovarian tissue. This overexpression tended to be higher in tumors from late stage disease than from early stage disease. The histochemical studies suggest that hK6 is synthesized by the epithelial cells of the ovary and is distributed diffusely within the cytoplasmic compartment.

Epithelial ovarian cancer has one of the worst prognoses among gynecologic malignancies, largely because over three-quarters of the diagnoses are made at a time when the disease has already established regional or distant metastases (33). Compounding the problem, tumor progression and aggressiveness correlate variably with conventional clinical and pathological markers. Thus there is an important need for additional diagnostic and prognostic markers for this disease and a number of potential markers have been identified.

Below full citations are set out for the references referred to in the specification.

**Table 1: Analysis of hK6 protein in various fluids**

| Sample | hK6, µg/M | | | N² | Positivity rate |
|---|---|---|---|---|---|
| | Range | Mean (SD) | Median | | (%) |
| Milk¹ | 398-7,638 | 2,588 (1,607) | 2,531 | 20 | 100 |
| Cerebrospinal fluid (CSF) | 41-2,053 | 605 (485) | 525 | 21 | 100 |
| NAF (normal)³ | - | 914 | - | 1 (pool) | 100 |
| NAF (cancer)⁴ | - | 737 | - | 1 (pool) | 100 |
| Breast cyst fluid | 34-97 | 74 (25) | 84 | 5 (pools) | 100 |
| Male serum | 2.0 -12.6 | 6.9 (2.6) | 6.7 | 18 | 100 |
| Female serum | 0 - 8.1 | 4.1 (2.0) | 4.4 | 18 | 100 |
| Seminal plasma | 0-17.7 | 6.8 (5.5) | 5.0 | 16 | 81 |
| Amniotic fluid | 0-9.5 | 1.1 (2.2) | 0 | 21 | 33 |
| Breast tumor cytosols | 0 - 33 | 2.1 (7.0) | 0 | 36 | 17 |
| Urine | 0 | 0 | 0 | 10 | 0 |

| | | | | | |
|---|---|---|---|---|---|
| 1. From lactating women 2. Number of samples tested 3. Nipple aspirate fluid 4. NAF obtained from patients with breast cancer | | | | | |

**Table 2 Concentration of human kallikrein 6 (hk6) in scrum of normal individuals and patients with various malignancies.**

| | | hK6, µg/L | | | | |
|---|---|---|---|---|---|---|
| | | | | | | |
| **Patient Group** | **Number of Samples** | **Min** | **Max** | **Median** | **95^{th} Percentile** | **Number of Patients with hK6 > 15µg/L (%)** |
| Normal males | 41 | 3.2 | 11.4 | 7.5 | 11.1 | 0 (0) |
| Normal females | 40 | 3.5 | 13.7 | 7.0 | 10.8 | 0 (0) |
| Breast cancer¹ | 24 | 1.1 | 11.9 | 4.3 | 9.7 | 0 (0) |
| Medullary thyroid carcinoma² | 29 | 0 | 13.9 | 5.0 | 11.8 | 0 (0) |
| Testicular cancer³ | 78 | 2.0 | 32.2 | 9.3 | 14.3 | 1 (2)* |
| Gastrointestinal cancer⁴ | 28 | 2.6 | 10.6 | 5.7 | 9.6 | 0(0) |
| Prostate cancer⁵ | 40 | 1.0 | 16.1 | 4.1 | 9.5 | 1 (2)** |
| Lung cancer | 18 | 2.6 | 7.4 | 5.2 | 6.7 | 0 (0) |
| Ovarian cancer⁶ | 80 | 1.0 | 206 | 23.0 | 148 | 53(66) |

| | | | | | | |
|---|---|---|---|---|---|---|
| 1. With serum C.A 15.3 levels ≥ 414 KU/L (upper ref. range 35 KU/L). 2. With calcitonin levels ≥ 1,135 ng/L (upper ref. range 100 ng/L). 3. With hCG levels ≥ 69 IU/L (upper ref. range 10 IU/L) or AFP levels ≥ 110 µg/L (upper ref. range 10 µg/L). 4. With CA 19.9 levels ≥ 629 KU/L (upper ref. range 37 KU/L) and CEA levels ≥ 1.000 µg/L (upper ref. range 5 µg/L). 5. With PSA ≥ 324 µg/L (upper ref range 4 µg/L). 6. With CA 125 ≥ 372 KU/L (upper ref. range 35 KU/L). | | | | | | |

**Table 3: Descriptive statistics of serum hK6 in non-cancer (healthy), benign disease and ovarian cancer patients.**

| | | | **Percentiles** | | | | |
|---|---|---|---|---|---|---|---|
| **Variable** | **Mean ± SE^{a}** | **Range** | **5** | **25** | **50** | **75** | **95** |
| Non-Cancer(N=97) | | | | | | | |
| hK6 (µg/L) | 2.94 ± 0.099 | 0.89 - 6.58 | 1.49 | 2.28 | 2.90 | 3.54 | 4.44 |
| | | | | | | | |
| Benign Disease (N = 141) | 3.12 ± 0.074 | 1.30 - 6.16 | 1.99 | 2.50 | 3.00 | 3.60 | 4.88 |
| hK6 (µg/L) | | | | | | | |
| | | | | | | | |
| Pre-Surgical Ovarian Cancer (N=146) | | | | | | | |
| hK6 (µg/L) | 6.81 ± 0.57 | 1.30 - 38.00 | 2.19 | 3.12 | 4.40 | 7.15 | 25.06 |
| | | | | | | | |
| Post-Surgical Ovarian Cancer (N=105) | | | | | | | |
| hK6(µg/L) | 3.87 ± 0.25 | 0.80-21.82 | 1.82 | 2.66 | 3.20 | 4.20 | 7.72 |

| | | | | | | | |
|---|---|---|---|---|---|---|---|
| ^{a} Standard error | | | | | | | |

**Table 4: Comparison of sensitivity and specificity of serum hK6 concentration at selected cut-off points**

| **Parameter** | **Cut-Off** | **Sensitivity** | **Specificity** |
|---|---|---|---|
| | | **(%)** | **(%)** |
| Total population (N = 384) | 2.20 | 95 | 19 |
| hK6 (µg/L)) | 2.50 | 90 | 29 |
| | | | |
| | 4.20 | 54 | 90 |
| | 4.40 | 50 | 95 |
| | | | |
| CA 125 < 23 KU/L (N = 182) | 2.27 | 95 | 24 |
| hK6 (µg/L) | 2.40 | 90 | 28 |
| | | | |
| | 4.30 | 17 | 90 |
| | 4.40 | 13 | 95 |
| | | | |
| CA125 23-60 KU/L (N = 65) | 2.20 | 95 | 10 |
| hK6 (µg/L) | 2.40 | 90 | 19 |
| | | | |
| | 4.00 | 26 | 90 |
| | 4.20 | 15 | 95 |
| | | | |
| CA 125 > 60 KU/L (N = 110) | 2.20 | 95 | 16 |
| hK6 (µg/L) | 2.70 | 90 | 43 |
| | | | |
| | 4.50 | 71 | 90 |
| | 5.56 | 65 | 95 |

**Table 5: Diagnostic sensitivities for ovarian cancer with CA125 alone, hK6 alone and CA125 + hK6 analysis at 90 and 95% specificity cut-offs for both markers**

| | **Sensitivity at 90% specificity** | **Sensitivity at 95% specificity** |
|---|---|---|
| *All patients with known stage (N=124)* | | |
| CA125 | 60 | 56 |
| hK6 | 58 | 53 |
| CA125+hK6 | 72 | 69 |
| | | |

| *Stage I*/*II patients (N=43)* | | |
|---|---|---|
| CA125 | 30 | 26 |
| hK6 | 26 | 21 |
| CA125+hK6 | 42 | 37 |

**Table 6: Relative risk^{a} (RR) of ovarian cancer according to quartiles of serum hK6**

| | **Quartiles (µg/L)** | | | |
|---|---|---|---|---|
| | | | | |
| **Parameter** | **1** | **2** | **3** | **4** |
| | (0.89-2.60) | (2.61-3.29) | (3.30-4.27) | (4.28-38.00) |
| | n = 96 | n = 96 | n = 96 | n = 96 |
| hK6 unadjusted ^{a} | | | | |
| RR | 1.00 | 1.41 | 3.12 | 20.00 |
| 95 % confidence intervals | | 0.71-2.79 | 1.43-6.85 | 7.70-48.46 |
| p value | | 0.32 | 0.003 | < 0.001 |

| hK6 adjusted ^{b} | | | | |
|---|---|---|---|---|
| RR | 1.00 | 1.21 | 2.31 | 5.33 |
| 95% confidence intervals | | 0.56-2.62 | 1.05-5.02 | 2.32-12.24 |
| p value | | 0.62 | 0.036 | < 0.001 |

| | | | | |
|---|---|---|---|---|
| ^{a} Estimated from unconditional logistic regression models. ^{b} Multivariate models were adjusted with the CA125 quartiles. | | | | |

**Table 7: Relationship between hK6 status and other variables in ovarian cancer patients ***

| | | No. of patients (%) | | |
|---|---|---|---|---|
| **Variable** | **Patients** | **hK6 Negative** | **hK6 Positive** | **p Value** |
| **Stage** | | | | |
| I | 32 | 27 (84.4) | 5 (15.6) | |
| II | 11 | 8 (72.7) | 3(27.3) | < 0.001^{a} |
| III | 73 | 18 (24.7) | 55 (75.3) | |
| IV | 8 | 3 (37.5) | 5 (62.5) | |
| x | 22 | | | |

| **Grade** | | | | |
|---|---|---|---|---|
| G1 | 39 | 31 (79.5) | 8 (20.5) | |
| G2 | 24 | 7 (29.2) | 17 (70.8) | < 0.001^{a} |
| G3 | 62 | 19 (30.6) | 43 (69.4) | |
| x | 21 | | | |

| **Histotype** | | | | |
|---|---|---|---|---|
| Serous | 74 | 24 (32.4) | 50 (67.6) | |
| Endometrioid | 15 | 10 (66.7) | 5 (33.3) | < 0.001^{a} |
| Mucinous | 22 | 20 (90.9) | 2(9.1) | |
| Others | 27 | 17 (63.0) | 10 (37.0) | |
| x | 8 | | | |

| **Residual tumor (cm)** | | | | |
|---|---|---|---|---|
| 0 | 76 | 52 (68.4) | 24 (31.6) | |
| 1-2 | 17 | 3(17.6) | 14(82.4) | < 0.001^{a} |
| > 2 | 35 | 6(17.1) | 29(82.9) | |
| x | 18 | | | |

| **Debulking success^{c}** | | | | |
|---|---|---|---|---|
| SO | 49 | 9(18.4) | 40(81.6) | <0.001^{b} |
| OD | 81 | 53 (65.4) | 28 (34.6) | |
| x | 16 | | | |

| **Response to CTX^{d}** | | | | |
|---|---|---|---|---|
| NC/PD | 21 | 4 (19.0) | 17 (81.0) | <0.001^{b} |
| CR/PR | 107 | 61 (57.0) | 46 (43.0) | |
| NE | 18 | | | |

| | | | | |
|---|---|---|---|---|
| * hK6 cut-off = 4.4 µg/L (median) ^{a} χ² test ^{b} Fisher's Exact Test ^{c} OD, Optimal debulking (0-1 cm); SO, Suboptimal debulking (>1 cm) ^{d} CTX, chemotherapy; NC, no change; PD, progressive disease; CR, complete response; PR, partial response; NE, not evaluated x Status unknown. | | | | |

**Table 8: Univariate and multivariate analysis of serum hK6 in relation to progression-free and overall survival**

| | Progression-free survival | | | | Overall survival | | |
|---|---|---|---|---|---|---|---|
| Variable | | HR^{a} | 95% Cl^{b} | p Value | HR^{a} | 95% Cl^{b} | p Value |
| | | **Univariate analysis** | | | | | |
| **hK6** | negative | 1.00 | | | 1.00 | | |
| | positive | 4.10 | 2.28-7.36 | < 0.001 | 3.15 | 1.36-7.29 | 0.007 |
| as a continuous variable | | 1.068 | 1.041-1.095 | < 0.001 | 1.075 | 1.038-1.11 | < 0.001 |
| **CA125** | negative^{d} | 1.00 | | | 1.00 | | |
| | positive^{d} | 2.52 | 1.45-4.38 | 0.001 | 2.36 | 1.03-5.42 | 0.041 |
| as a continuous variable | | 1.001 | 1.000-1.002 | < 0.001 | 1.001 | 1.000-1.003 | 0.018 |
| **Grading** | (ordinal) | 2.50 | 1.71-3.64 | < 0.001 | 2.34 | 1.53-3.58 | < 0.001 |
| **Residual tumor** | (ordinal) | 1.23 | 1.13-1.34 | < 0.001 | 1.31 | 1.21-1.41 | < 0.001 |
| **Histologic type**^{c} | | 2.49 | 1.37-4.54 | 0.003 | 4.25 | 1.44-12.53 | < 0.008 |

| | | **Multivariate analysis** | | | | | |
|---|---|---|---|---|---|---|---|
| **hK6** | negative | 1.00 | | | 1.00 | | |
| | positive | 4.86 | 1.10-21.47 | 0.036 | 5.08 | 1.07-23.69 | 0.038 |
| as a continuous variable | | 1.047 | 1.007-1.089 | 0.019 | 1.063 | 1.007-1.12 | 0.025 |
| **CA125** | negative^{d} | 1.00 | | | 1.00 | | |
| | positive^{d} | 2.86 | 0.69-11.74 | 0.14 | 2.17 | 0.38-63.17 | 0.38 |
| **Stage of disease** | (ordinal) | 2.54 | 1.37-4.69 | 0.003 | 6.34 | 2.27-17.7 | < 0.001 |
| **Grading** | (ordinal) | 1.63 | 0.94-2.82 | 0.078 | 1.56 | 0.66-3.68 | 0.31 |
| **Residual tumor** | (ordinal) | 1.09 | 0.42-2.26 | 0.15 | 1.01 | 0.80-1.24 | 0.98 |
| **Histologic type**^{c} | | 1.08 | 0.75-1.56 | 0.65 | 1.18 | 0.94-1.31 | 0.18 |

| | | | | | | | |
|---|---|---|---|---|---|---|---|
| ^{a} Hazard ratio (HR) estimated from Cox proportional hazard regression model ^{b} Confidence interval of the estimated HR ^{c} Serous vs others ^{d} Cut-off = 98 KU/L (95% specificity; 53% sensitivity; 48^{th} percentile). | | | | | | | |

**Table 9. Relationship between hK6 status and other variables in 180 ovarian cancer patients.**

| | | No. of patients (%) | | |
|---|---|---|---|---|
| Variable | Patients | hK6 negative | hK6 positive | P value |
| Stage | | | | |
| I | 44 | 38 (86.4) | 6 (13.6) | |
| II | 13 | 8 (61.5) | 5 (38.5) | 0.034^{a} |
| III | 110 | 72 (65.4) | 38 (34.5) | |
| IV | 13 | 7 (53.8) | 6 (46.2) | |

| Grade | | | | |
|---|---|---|---|---|
| G1 | 25 | 21 (84.0) | 4 (16.0) | |
| G2 | 27 | 21 (77.8) | 6 (22.2) | 0.33^{a} |
| G3 | 119 | 84 (70.6) | 35 (29.4) | |
| x | 9 | | | |

| Histotype | | | | |
|---|---|---|---|---|
| Serous | 80 | 52 (65.0) | 28 (35.0) | |
| Undifferentiated | 27 | 17 (63.0) | 10 (37.0) | |
| Endometrioid | 32 | 27 (46.7) | 5 (53.3) | |
| Mucinous | 13 | 10 (76.9) | 3 (13.1) | 0.31^{b} |
| Clear cell | 14 | 11 (78.6) | 3 (21.4) | |
| Mullerian | 10 | 8 (80.0) | 2 (20.0) | |
| Others | 4 | 3 (75.0) | 1 (25.0) | |

| Residual tumor (cm) | | | | |
|---|---|---|---|---|
| 0 | 80 | 67 (83.2) | 13 (16.3) | |
| 1-2 | 29 | 16 (55.2) | 13(44.8) | 0.002^{a} |
| >2 | 64 | 40 (62.5) | 24 (37.5) | |
| x | 7 | | | |

| Menopause | | | | |
|---|---|---|---|---|
| Pre/peri | 50 | 32 (64.0) | 18 (36.0) | 0.075^{b} |
| Post | 130 | 99 (76.2) | 31 (23.8) | |

| Response to CTX^{c} | | | | |
|---|---|---|---|---|
| NC/PD | 15 | 11 (73.3) | 4 (26.7) | 0.99^{b} |
| CR/PR | 148 | 104 (70.3) | 44 (29.7) | |
| NE | 17 | | | |

| | | | | |
|---|---|---|---|---|
| ^{a} χ² test. ^{b} Fisher's Exact Test ^{c} CTX; chemotherapy, NC; no change, PD; progressive disease, CR; complete response, PR; partial response, NE; not evaluated. x. Status unknown. | | | | |

**Table 10. Univariate and Multivariate Analysis of Prognostic Value of hK6**

| | **Progression-free survival (PFS)** | | | **Overall survival (OS)** | | |
|---|---|---|---|---|---|---|
| **Variable** | | | | | | |
| | HR^{a} | 95% CI^{b} | P value | HR^{a} | 95% CI^{b} | P value |
| | ***Univariate Analysis*** | | | | | |
| **hK6** | | | | | | |
| Negative | 1.00 | | | 1.00 | | |
| Positive | 1.71 | 1.11-2.64 | 0.015 | 1.88 | 1.09-3.21 | 0.022 |
| as a continuous variable | 1.005 | 1.002-1.007 | 0.001 | 1.004 | 0.999-1.008 | 0.074 |
| | | | | | | |
| **Stage of disease** (ordinal) | 2.79 | 2.07-3.79 | <0.001 | 3.07 | 2.05-4.61 | <0.001 |
| **Grading** (ordinal) | 1.95 | 1.38-2.75 | <0.001 | 2.07 | 1.31-3.29 | 0.002 |
| **Residual tumor** (ordinal) | 1.27 | 1.20-1.34 | <0.001 | 1.31 | 1.22-1.41 | <0.001 |
| **Histologic type^{c}** | 0.83 | 0.68-1.00 | 0.055 | 0.88 | 0.69-1.13 | 0.34 |
| Age | 1.012 | 0.99-1.03 | 0.14 | 1.015 | 0.99-1.03 | 0.15 |

| | ***Multivariate Analysis*** | | | | | |
|---|---|---|---|---|---|---|
| **hK6** | | | | | | |
| Negative | 1.00 | | | 1.00 | | |
| Positive | 1.40 | 0.84-2.32 | 0.19 | 1.08 | 0.79-1.49 | 0.62 |
| as a continuous variable | 1.002 | 0.99-1.006 | 0.22 | 1.001 | 0.99-1.004 | 0.69 |
| | | | | | | |
| **Stage of disease** (ordinal) | 1.57 | 1.09-2.27 | 0.014 | 1.72 | 1.053-2.82 | 0.03 |
| **Grading** (ordinal) | 1.31 | 0.84-2.32 | 0.18 | 1.31 | 0.75-2.25 | 0.33 |
| **Residual tumor** (ordinal) | 1.14 | 1.05-1.24 | 0.001 | 1.21 | 1.09-1.34 | <0.001 |
| **Histologic type^{c}** | 0.95 | 0.82-1.11 | 0.57 | 1.04 | 0.86-1.26 | 0.68 |
| **Age** | 1.02 | 0.99-1.039 | 0.12 | 1.02 | 0.99-1.04 | 0.21 |

| | | | | | | |
|---|---|---|---|---|---|---|
| ^{a} Hazard ratio (HR) estimated from Cox proportional hazard regression model ^{b} Confidence interval of the estimated HR. ^{c} Serous vs. others | | | | | | |

**Table 11. Cox proportional hazard regression analysis for subgroups of patients**

| | **Progression-free survival** | | | **Overall survival** | | |
|---|---|---|---|---|---|---|
| **Variable** | HR^{a} | 95% Cl^{b} | P value | HR^{a} | 95% Cl^{b} | P value |
| **Tumor grade I-II** | | | | | | |
| hK6 univariate | 9.25 | 3.33-25.67 | <0.001 | 5.05 | 1.63-15.71 | 0.005 |
| hK6 multivariate^{c} | 4.29 | 1.17-15.65 | 0.027 | 4.05 | 1.23-16.6 | 0.023 |

| **Tumor grade III** | | | | | | |
|---|---|---|---|---|---|---|
| hK6 univariate | 1.45 | 0.87-2.39 | 0.14 | 1.69 | 0.91-3.14 | 0.091 |
| hK6 multivariate^{c} | 1.03 | 0.58-1.83 | 0.91 | 1.02 | 0.48-2.13 | 0.96 |

| **Stage I-II** | | | | | | |
|---|---|---|---|---|---|---|
| hK6 univariate | 0.90 | 0.18-4.35 | 0.89 | 1.49 | 0.13-16.53 | 0.74 |
| hK6 multivariate^{d} | 1.83 | 0.17-19.41 | 0.61 | 2.23 | 0.20-25.04 | 0.51 |

| **Stage III-IV** | | | | | | |
|---|---|---|---|---|---|---|
| hK6 univariate | 2.04 | 1.26-3.29 | 0.004 | 1.98 | 1.12-3.47 | 0.017 |
| hK6 multivariate^{d} | 1.57 | 0.93-2.68 | 0.092 | 1.33 | 0.71-2.53 | 0.37 |

| **Optimal debulking success**^{e} | | | | | | |
|---|---|---|---|---|---|---|
| hK6 univariate | 1.81 | 0.72-4.55 | 0.20 | 2.61 | 0.70-9.73 | 0.15 |
| hK6 multivariate^{f} | 3.75 | 1.39-10.09 | 0.019 | 5.57 | 1.47-21.04 | 0.011 |

| **Suboptimal debulking success**^{e} | | | | | | |
|---|---|---|---|---|---|---|
| hK6 univariate | 1.39 | 0.83-2.32 | 0.20 | 1.16 | 0.64-2.09 | 0.62 |
| hK6 multivariate^{f} | 1.27 | 0.72-2.23 | 0.40 | 1.19 | 0.62-2.27 | 0.59 |

| | | | | | | |
|---|---|---|---|---|---|---|
| ^{a} Hazard ratio (HR) estimated from Cox proportional hazard regression model ^{b} Confidence interval of the estimated HR. ^{c} Multivariate models were adjusted for stage of disease, residual tumor, histologic type and age. ^{d} Multivariate models were adjusted for tumor grade, residual tumor, histologic type and age. ^{e} Optimal debulking (0 - 1 cm residual tumor); suboptimal debulking (> 1 cm residual tumor) ^{f} Multivariate models were adjusted for stage of disease, tumor grade, histologic type and age. | | | | | | |

### FULL CITATIONS FOR REFERENCES REFERRED TO IN THE SPECIFICATION

1. Diamandis EP, Yousef GM, Luo LY, Magklara A, Obiezu CV. The new human kallikrein gene family: implications in carcinogenesis. Trends Endocrinol Metab 2000;11:54-60.
2. Diamandis EP. Prostate specific antigen - its usefulness in clinical medicine. Trends Endocrinol Metab 1999;25:14-16.
3. McCormack RT, Rittenhouse HG, Finlay JA, Sokoloff RL, Wang TJ, Wolfert RL, Lilja H, Oesterling JE. Molecular forms of prostate-specific antigen and the human kallikrein gene family: a new era. Urology 1995;45:729-744.
4. Chu TM. Prostate-specific antigen and early detection of prostate cancer. Tumor Biol 1997;18:123-134.
5. Stenman U-H. New ultrasensitive assays facilitate studies on the role of human glandular kallikrein (hK2) as a marker for prostatic disease. Clin Chem 1999;45:753-754.
6. Rittenhouse HG, Finlay JA, Mikolajczyk SD, Partin AW. Human kallikrein 2 (hK2) and prostate-specific antigen (PSA): Two closely related, but distinct, kallikreins in the prostate. Crit Rev Clin Lab Sci 1998;35:275-368.
7. Little SP, Dixon EP, Norris F, Buckley W, Becker GW, Johnson M, Dobbins JR, et al. Zyme, a novel and potentially amyloidogenic enzyme cDNA isolated from Alzheimer;s disease brain. J Biol Chem 1997;272:25135-25142.
8. Anisowicz A, Sotiropoulou G, Stenman G. Mok SC, Sager R. A novel protease homolog differentially expressed in breast and ovarian cancer. Mol Med 1996;2:624-636.
9. Yamashiro K, Tsuruoiko N, Kodama S, Tsujimoto M, Yamamura T, Tanaka T, Nakazato H, Yamaguchi N. Molecular cloning of a novel trypsin-like serine protease (neurosin) preferentially expressed in brain. Biochim Biophys Acta 1997;1350:11-14.
10. Diamandis EP, Yousef GM, Clements J, Ashworth LK, Yoshida S, Egelrud T, Nelson PS, Shiosaka S, Little S, Lilja H, Stenman U-H, Rittenhouse HG, Wain H. New nomenclature for the human tissue kallikrein gene family. Clin Chem (in press).
11. Yousef GM, Luo L-Y, Scherer SW, Sotiropoulou G, Diamandis EP. Molecular characterization of zyme/proteaseM/neurosin (PRSS9), a hormonally regulated kallikrein-like serine protease. Genomics 1999; 62: 251-259.
12. Diamandis EP, Yousef GM, Soosaipillai AR, Grass L, Porter A, Little S, Sotiropoulou G. Immunofluorometric assay of human kallikrein 6 (zyme/protease M/neurosin) and preliminary clinical applications. Clin Biochem.200, 33: 369-375.
13. Riman T, Persson I, Staffan N. Hormonal aspects of epithelial ovarian cancer: review of epidemiological evidence. Clin Endocrinol 1998;49:695-707.
14. National Cancer Institute of Canada: Canadian Cancer Statistics 1999, Toronto, Canada, 1999.
15. Menon U, Jacobs IJ. Recent developments in ovarian cancer screening. Curr Opin Obstet Gynecol 2000;12:39-42.
16. Davellar EM, van Kamp GJ, Verstraeten RA, Kenemans P. Comparison of serum immunoassays for the quantification of CA 125 antigen in serum. Clin Chem 1998;44:1417-1422.
17. Rosenthal AN, Jacobs IJ. The role of CA 125 in screening for ovarian cancer. Int J Biol Markers 1998;13:216-220.
18. Maggino T, Gadducci A. Serum markers as prognostic factors in epithelial ovarian cancer: an overview. Eur J Gynaecol Oncol 2000;21:64-69.
19. Bast RC Jr, Xu FJ, Yu YH, Barnhill S, Zhang Z, Mills GB. CA 125: the past and the future. Int J Biol Markers 1998;13:179-187.
20. Robertson DM, Kahir N, Burger HG, Mamers P, McCloud PI, Pettersson K, MCGuckin M. Combined inhibin and CA 125 assays in the detection of ovarian cancer. Clin Chem 1999;45:651-658.
21. Lambert-Messerlian GM. Is inhibin a serum marker for ovarian cancer? Eur J Endocrinol 2000;42:331-333.
22. Ala-Fossi SL, Maenpaa J, Blauer M, Tuohimaa P, Punnonen R. Inhibin A, B and pro-alphaC in serum and peritoneal fluid in postmenopausal patients with ovarian tumors. Eur J Endocrinol 2000;142:334-339.
23. Burger HG, Baillie A, Drummond AE, Healy DL, Jobling T, Mamers P, Robertson DM, et al. Inhibin and ovarian cancer. J Reprod Immunol 1998;39:77-87.
24. Xu FJ, Yu YH, Daly L, DeSombre K, Anselmino L, Hass GM, Berchuck A, et al. OVX1 radioimmunoassay complements CA-125 for predicting presence of residual ovarian carcinoma at second-look surgical surveillance procedures. J Clin Oncol 1993;11:1506-1510.
25. Berek JS, Bast RC Jr. Ovarian cancer screening. The use of serial complementary tumor markers to improve sensitivity and specificity for early detection. Cancer 1995;76:2092-2096.
26. Xu Y, Shen Z, Wiper DW, Wu M, Morton RE, Elson P, Kennedy AW, et al. Lysophosphatidic acid as a potential biomarker for ovarian and other gynecologic markers. J Am Med Assoc 1998;280:7190723.
27. Myer, t, Rustin GJS. Role of tumour markers in monitoring epithelial ovarian cancer. Br J Cancer 2000;82:1535-1538.
28. Woolas RP, Xu FJ, Jacobs IJ, Yu Y, Daly L, Berchuck A, et al. Elevation of multiple serum markers in patients with stage I ovarian cancer. J. Natl Cancer Inst 1993;85:1748-1751.
29. Woolas RP, Conaway MR, Xu F, Jacobs IJ Yu Y, Daly, L et al. Combinations of multiple serum markers are superior to individual assays for discriminating malignant from benign pelvic masses. Gynecol Oncol 1995;59:111-116.
30. Cox, DR. Regression tables and life tables. J. R. Stat.Cos.B, 34:187-202,1972.
31. Kaplan, E.L, and Meier, P. Nonparametric estimation from incomplete observations. J. Am. Stat. Assoc. 53:457-481, 1958.
32. Mantel, N. Evaluation of survival data and two new rank order statistics arising in its consideration. Cancer Chemother.Rep. 50:163-170, 1966.
33. Gatta, G. Lasota, M.B. and Verdecchia, A. Survival of European women with gyneacological tumours during the period 1978-1989. Eur. J. Cancer, 34: 2218-2225, 1998.
34. Obiezu, CV et al, Clin Cancer Res. 2001;7:2380-2386.
35. Kim, H, et al, Br. J. Cancer 2001;8:643-650.
36. Magklara, A., et al., Clin.Cancer Res 2001;7:806-811.
37. Luo, LY et al, Clin Cancer Res 22001;7:2372-2379.
38. Luo, LY et al, Clin Chim Acta 2001;306:111-118.
39. Dong Y, et al, Clin Cancer Res 2001;7:2363-2371.
40. Tanimoto H. et al, Cancer 1999;86:2074-2082.
41. Underwood LJ et al Cloning of tumor-associated differentially expressed gene-14, a novel serine protease overexpressed by ovarian carcinoma
42. Shigemasa K et al J Soc Gynecol Investig 200;7:357-362.
43. Tanimoto, H., et al, Cancer Res 1997;57:2884-2887.
44. Hirahara F., et al Gynecol Oncol 1998;68:162-165.
45. Duffy, MJ, Clin Exp Metasis 1992;10:145-155.
46. Matrisian, LM, Curr Biol 1999;9:R776-R778.
47. Kleiner DE, Stetler-Stevenson WG, Cancer Chemother Pharmacol 1999;43:S42-S51.
48. Woodhouse EC et al, Cancer 1997;80:1529-2537.
49. Aznavoorian, S et al Cancer 1993;71:1368-1383.

## Claims

1. An *in vitro* method for diagnosing or monitoring ovarian cancer or determining the prognosis of ovarian cancer in a subject comprising:
(a) determining the level of human kallikrein 6 in a serum sample from the subject; and
(b) comparing the determined level of human kallikrein 6 to a level present in a control serum sample acquired from a control subject known not to be inflicted by ovarian cancer or with benign disease or a control serum sample taken from the subject at a different time, wherein an increase in the level of human kallikrein 6 in the serum sample from the subject as compared to the level of human kallikrein 6 in the control serum sample is indicative of ovarian cancer in the subject or poor prognosis.

2. A method as claimed in claim 1 comprising in step (a):
(i) contacting the serum sample with an antibody specific for human kallikrein 6 which is directly or indirectly labelled with a detectable substance;
(b) detecting the detectable substance to determine the level of human kallikrein 6 in the sample.

3. A method as claimed in claim 2 which further comprises:
in step (a), further contacting the serum sample with a second antibody specific for hK6 which is immobilized;
following step (a), separating the first antibody from the second antibody to provide a first antibody phase and second antibody phase;
in step (b), detecting the detectable substrate in either the first or second antibody phase thereby determining the level of human kallikrein 6 in the biological sample.

4. A method as claimed in claim 2 wherein in step (a) the first and second antibodies are contacted simultaneously or sequentially with the biological, sample.

5. A method as claimed in any one of claims 2 to 4 wherein the antibody is a monoclonal antibody, a polyclonal antibody, immunologically active antibody fragments, humanized antibody, an antibody heavy chain, an antibody light chain, a genetically engineered single chain Fᵥ molecule, or a chimeric antibody.

6. A method as claimed in any one of claims 2 to 5 wherein the detectable substance is alkaline phosphatase.

7. A method as claimed in claim 6 wherein the alkaline phosphatase is detected using a fluorogenic substrate.

8. A method as claimed in any of the preceding claims wherein the level of human kallikrein 6 is determined using time-resolved fluorescence.

9. A method as claimed in any one of the preceding claims which further comprises determining levels of CA125 in the serum sample.

10. A method as claimed in any one of the preceding claims for monitoring of the subject after primary treatment.

11. A method as claimed in any one of claims 1 to 8 for determining the prognosis of ovarian cancer disease in the subject wherein a level of greater than 4.4µg/L indicates a significantly worse prognosis.

12. A method as claimed in any one of claims 1 to 9, wherein determining the prognosis comprises predicting the outcome of surgery, the response to chemotherapy or the survival chances of the subject.

13. A method as claimed in any one of the preceding claims wherein detection of a level of human kallikrein 6 in the serum sample from the subject greater than the level determined in the serum sample from the control subject indicates late stage disease, or an increased risk of disease progression and mortality.

## Patentansprüche

1. *In vitro -* Verfahren zum Diagnostizieren oder Überwachen von Eierstockkrebs oder zum Bestimmen der Prognose für Eierstockkrebs in einem Subjekt, wobei man
(a) die Menge an humanem Kallikrein 6 in einer Serumprobe von dem Subjekt bestimmt und
(b) die bestimmte Menge an humanem Kallikrein 6 mit einer Menge vergleicht, die in einer Kontroll-Serumprobe vorhanden ist, welche man von einem Kontroll-Subjekt erhalten hat, von dem bekannt ist, daß es nicht von Eierstockkrebs oder einer benignen Erkrankung befallen ist, oder in einer Kontroll-Serumprobe, die von dem Subjekt zu einem anderen Zeitpunkt genommen wurde, wobei eine Zunahme der Menge an humanem Kallikrein 6 in der Serumprobe von dem Subjekt im Vergleich zu der Menge an humanem Kallikrein 6 in der Kontroll-Serumprobe ein Hinweis für Eierstockkrebs in dem Subjekt oder für eine schlechte Prognose ist.

2. Verfahren nach Anspruch 1, welches in Stufe (a) folgendes umfaßt:
(i) in Kontakt bringen der Serumprobe mit einem für humanes Kallikrein 6 spezifischen Antikörper, welcher direkt oder indirekt mit einer detektierbaren Substanz markiert ist,
(b) Feststellen der detektierbaren Substanz zur Bestimmung der Menge an humanem Kallikrein 6 in der Probe.

3. Verfahren nach Anspruch 2, welches weiterhin folgendes umfaßt:
In Stufe (a) weiterhin das in Kontakt bringen der Serumprobe mit einem für hK6 spezifischen zweiten Antikörper, welcher immobilisiert ist,
nach Stufe (a) das Abtrennen des ersten Antikörpers von dem zweiten Antikörper, um eine Phase des ersten Antikörpers und eine Phase des zweiten Antikörpers zu erhalten,
in Stufe (b) das Feststellen des detektierbaren Substrates in der Phase entweder des ersten oder des zweiten Antikörpers, wobei die Menge an humanem Kallikrein 6 in der biologischen Probe bestimmt wird.

4. Verfahren nach Anspruch 2, wobei in Stufe (a) die ersten und zweiten Antikörper gleichzeitig oder nacheinander mit der biologischen Probe in Kontakt gebracht werden.

5. Verfahren nach einem der Ansprüche 2 bis 4, wobei der Antikörper eine monoklonaler Antikörper, ein polyklonaler Antikörper, immunologisch aktive Antikörperfragmente, humanisierter Antikörper, eine schwere Kette eines Antikörpers, eine leichte Kette eines Antikörpers, ein genetisch hergestelltes Einzelketten-Fᵥ - Molekül oder ein chimarer Antikörper ist/sind.

6. Verfahren nach einem der Ansprüche 2 bis 5, wobei die detektierbare Substanz alkalische Phosphatase ist.

7. Verfahren nach Anspruch 6, wobei die alkalische Phosphatase unter Verwendung eines fluorogenen Substrates detektiert wird.

8. Verfahren nach einem der vorangegangenen Ansprüche, wobei die Menge an humanem Kallikrein 6 unter Anwendung von zeitaufgelöster Fluoreszenz bestimmt wird.

9. Verfahren nach einem der vorangegangenen Ansprüche, welches weiterhin das Bestimmen der Mengen von CA125 in der Serumprobe umfaßt.

10. Verfahren nach einem der vorangegangenen Ansprüche zur Überwachung des Subjekts nach einer ersten Behandlung.

11. Verfahren nach einem der Ansprüche 1 bis 8 zum Bestimmen der Prognose einer Eierstockkrebserkrankung in dem Subjekt, wobei eine Menge von mehr als 4,4µg/l eine signifikant schlechte Prognose anzeigt.

12. Verfahren nach einem der Ansprüche 1 bis 9, wobei die Bestimmung der Prognose die Vorhersage über den Ausgang einer chirurgischen Behandlung, über das Ansprechen auf Chemotherapie oder über die Überlebenschancen des Subjekts umfaßt.

13. Verfahren nach einem der vorangegangenen Ansprüche, wobei das Feststellen einer Menge von humanem Kallikrein 6 in der Serumprobe von dem Subjekt von mehr als der Menge, die in der Serumprobe von dem Kontrollsubjekt bestimmt wird, eine Erkrankung im späten Stadium oder ein erhöhtes Risiko für ein Fortschreiten der Erkrankung und Mortalität anzeigt.

## Revendications

1. Méthode *in vitro* pour le diagnostic ou le contrôle du cancer des ovaires ou la détermination du pronostic du cancer des ovaires chez un sujet, comprenant les étapes consistant à :
(a) déterminer le taux de kallikréine humaine 6 dans un échantillon de sérum provenant du sujet; et
(b) comparer le taux déterminé de kallikréine humaine 6 avec un taux présent dans un échantillon de sérum témoin obtenu à partir d'un sujet témoin connu comme n'étant pas atteint par le cancer des ovaires ou bien présentant une maladie bénigne ou un échantillon de sérum témoin prélevé chez le sujet à une période de temps différente, dans laquelle une augmentation du taux de kallikréine humaine 6 dans l'échantillon de sérum provenant du sujet, par comparaison avec le taux de kallikréine humaine 6 dans l'échantillon de sérum témoin, est une indication de la présence d'un cancer des ovaires chez le sujet ou d'un mauvais pronostic.

2. Méthode suivant la revendication 1, comprenant, dans l'étape (a) :
(i) la mise en contact de l'échantillon de sérum avec un anticorps spécifique de la kallikréine humaine 6 qui est marqué directement ou indirectement avec une substance détectable ;
(b) la détection de la substance détectable pour déterminer le taux de kallikréine humaine 6 dans l'échantillon.

3. Méthode suivant la revendication 2, qui comprend en outre :
dans l'étape (a), en outre, la mise en contact de l'échantillon de sérum avec un second anticorps spécifique de hK6 qui est immobilisé ;
après l'étape (a), la séparation du premier anticorps du second anticorps pour fournir une première phase d'anticorps et une seconde phase d'anticorps;
dans l'étape (b), la détection du substrat détectable dans la première ou seconde phase d'anticorps, ce qui permet de déterminer le taux de kallikréine humaine 6 dans l'échantillon biologique.

4. Méthode suivant la revendication 2, dans laquelle, dans l'étape (a), les premier et second anticorps sont mis en contact de manière simultanée ou séquentielle avec l'échantillon biologique.

5. Méthode suivant l'une quelconque des revendications 2 à 4, dans laquelle l'anticorps est un anticorps monoclonal, un anticorps polyclonal, un fragment d'anticorps immunologiquement actif, un anticorps humanisé, une chaîne lourde d'anticorps, une chaîne légère d'anticorps, une molécule Fᵥ monocaténaire modifiée génétiquement ou un anticorps chimère.

6. Méthode suivant l'une quelconque des revendications 2 à 5, dans laquelle la substance détectable est la phosphatase alcaline.

7. Méthode suivant la revendication 6, dans laquelle la phosphatase alcaline est détectée en utilisant un substrat fluorogène.

8. Méthode suivant l'une quelconque des revendications précédentes, dans laquelle le taux de kallikréine humaine 6 est déterminé en utilisant la fluorescence à résolution dans le temps.

9. Méthode suivant l'une quelconque des revendications précédentes, qui comprend en outre la détermination des taux de CA125 dans l'échantillon de sérum.

10. Méthode suivant l'une quelconque des revendications précédentes, pour le contrôle du sujet après le traitement primaire.

11. Méthode suivant l'une quelconque des revendications 1 à 8, pour la détermination du pronostic de la maladie consistant en le cancer des ovaires chez le sujet, dans laquelle un taux supérieur à 4,4 µg/l indique un pronostic très mauvais.

12. Méthode suivant l'une quelconque des revendications 1 à 9, dans laquelle la détermination du pronostic comprend la prévision du résultat d'une intervention chirurgicale, de la réponse à la chimiothérapie ou des probabilités de survie du sujet.

13. Méthode suivant l'une quelconque des revendications précédentes, dans laquelle la détection d'un taux de kallikréine humaine 6 dans l'échantillon de sérum du sujet supérieur au taux déterminé dans l'échantillon de sérum provenant du sujet témoin indique une maladie à un stade tardif, ou un risque accru de progression de la maladie et de mortalité.
